# EUROPEAN PATENT SPECIFICATION

(11) **EP 0 353 851 B1**
(45) Date of publication and mention of the grant of the patent: **19.01.1994**
(21) Application number: 89306326.3
(22) Date of filing: 22.06.1989
(51) Int. Cl.: C12N 15/86, C12N 7/01, C12N 5/10

(54) **Fowlpox virus non-essential regions**
Nichtessentielle Geflügelpockenvirus-Regionen
Régions non essentielles du virus du fowlpox

(30) Priority: 24.06.1988 GB 8815098; 27.02.1989 GB 8904411; 31.03.1989 GB 8907374; 08.05.1989 GB 8910520
(43) Date of publication of application: 07.02.1990
(73) Proprietor: BRITISH TECHNOLOGY GROUP LIMITED, London SE1 6BU (GB)
(72) Inventor: Binns, Matthew KcKinley, Huntingdon Cambridgeshire PE17 3NJ (GB); Boursnell, Michael Edward Griffith, Huntingdon Cambridgeshire PE17 6EF (GB); Campbell, Joan Iyabo Amiemenoghena, Huntingdon Cambridgeshire PE17 4TT (GB); Tomley, Fiona Margaret, Cambridge CB2 1LR (GB)
(74) Representative: Percy, Richard Keith

(56) References cited:
- EP-A- 0 284 416
- EP-A- 0 308 220
- WO-A-86/00528
- WO-A-89/03429

## Description

### Background of the invention

### 1. Field of the invention

This invention is in the field of recombinant DNA technology and relates to fowlpox virus as a vector for foreign DNA.

### 2. Description of the prior art

Several viruses with DNA genomes have been used to carry and express genes from other viruses or other species. Viruses used in such a way are known as 'vectors' and genes, other than their own, expressed in such a way are referred to as 'foreign genes'. One of the primary requirements for a virus to be used as a vector in this manner is a suitable site for insertion of the foreign gene. If insertion of a gene into a site in the virus causes disruption of some function essential for growth, then such a site could not be used. Suitable sites are those at which an insertion does not disrupt any function or those whose functions are not essential for viral growth and therefore can be disrupted with impunity. Such sites are known as 'non-essential regions'. The phrase 'non-essential' in this context means non-essential for growth under at least some conditions in which the virus can be grown in vitro and under at least some conditions in which it survives in vivo.

Examples of viruses which have been used as vectors by virtue of the fact that they contain non-essential regions are orthopoxviruses, adenoviruses and herpesviruses, although the regions used may be different in each case. Vaccinia virus (VV), which has been used as a vector, is an orthopoxvirus, a member of the pox virus family. Fowlpoxvirus (FPV), the subject of this patent application, is also a pox virus, but is a member of a different genus, the avipoxviruses. VV can be grown in tissue culture, and foreign genes can be inserted into the viral genome during this process. Several regions of VV have been found to be non-essential in vitro in more than one tissue-culture system. These include: large regions towards the left hand end, B. Moss et al., J. Virology 40, 387-395, (1981) who describe a mutant VV having a deletion 6.4 megaDaltons from the left-hand end; D. Panicali et al., J. Virology 37 1000-1010, 1981) who describe a mutant VV having a deletion starting 6.85 megaDaltons from the left-hand end; the thymidine kinase (TK) gene, D. Panicali and E. Paoletti, Proc. Natl. Acad. Sci. USA 79, 4927-4931 (1982); M. Mackett et al., J. Gen. Virol. 45, 683-701, (1982); and the vaccinia growth factor (VGF) gene, R.M.L. Buller et al., J. Virology 62, 866-874 (1988). Sites such as these might also be non-essential for growth in vivo. However in the case of both the TK and VGF gene, it has been found that although growth in tissue culture is unaffected or only slightly affected by insertion into the gene, growth and virulence in vivo are markedly affected, R.M.L. Buller et al., Nature 317, 813-815 (1985) and loc. cit. (1988), showing that these genes are not completely non-essential for growth of the virus in vivo. This in vivo attenuation may however be useful if it reduces unwanted pathogenic effects of the virus and accordingly growth in vivo with accompanying attenuation is a valid growth condition for the purpose of this invention.

Some sites are essential in some tissue culture systems and non-essential in others. For example there is a gene in VV which is essential for replication in human cells but which is non-essential in chicken embryo fibroblast cells, S. Gillard et al., Proc. Natl. Acad. Sci. USA 83, 5573-5577 (1986). Another gene in the related orthopoxvirus cowpox virus is essential for growth in chinese hamster ovary cells but not for growth in chicken embryo fibroblast cells, D. Spehner et al., J. Virology 62, 1297-1304 (1988). These examples show that differences in the tissue culture systems can affect which regions are non-essential.

The VV genome is far from being completely mapped and relatively little has been published about the FPV genome. It is known that FPV has a TK gene which has about 60% homology with the VV TK gene at the amino acid level, D.B. Boyle et al., Virology 156, 355-365 (1987). Like the VV TK gene, it serves as a non-essential region for homologous recombination under at least some conditions : see PCT Application WO 88/02022 (CSIRO). Using the E. coli xanthine-guanine phosphoribosyl transferase (Ecogpt) gene as a dominant selectable marker, in conjunction with the VV "7.5" promoter, the influenza haemagglutinin (HA) gene was inserted into a TK region of FPV and the FPV grown in chicken embryo skin cells. Expression of the HA gene was demonstrated by the binding of HA antibodies and labelled protein A to plaques of recombinant FPV.

The genome of FPV is known to have another similarities to VV in some regions. For example the DNA polymerase genes are closely related, having 42% homology at the amino acid level, M.M. Binns et al., Nucleic Acids Research 15, 6563-6573 (1987). On the other hand, F.M. Tomley, in a talk at the 6th Workshop on Poxvirus/Iridovirus, Cold Spring Harbor, New York, 24-28 September 1986, described an attempted correlation of an 11.2 kb fragment of FPV DNA, located near one end of the genome, with VV DNA. While she reported 25% amino acid homology between a gene predicitng a 48 kd polypeptide in FPV and a gene in VV coding for a 42 kd polypeptide, no other match of any significance was found. More detail of this work is given in F.M. Tomley et al., J. Gen. Virol 69, 1025-1040 (1988).

F.M. Tomley et al, quoting M. Mackett and L.C. Archard, J. General Virology 45, 683-701 (1979), also state at page 1038 that the terminal portions of orthopoxvirus genomes are less well conserved than the central region and also say that the terminal regions, of around 30 to 35 kb in length, are thought to encode genes which determine specific virus characteristics such as host range, virulence, tissue tropism and cytopathogenicity and are thus less likely to be conserved.

The terminal region of VV is known to be of possible interest in relation to non-essential regions. Thus, M.E. Perkus et al., Virology 152, 285-297 (1986), have found that deletions in the VV genome occur in the presence of bromoxydeuridine, one of which extended from about nucleotides 2700 to 24100 from the left-hand end and that the deletion mutant virus is viable in tissue culture (cell type not clearly stated).

Some information has been released about the very terminal region of FPV, in a poster by J.I.A. Campbell displayed at the International Poxvirus Workshop, Cold Spring Harbor, New York, 9-13 September 1987. (The poster gives more information than does the abstract published in "Modern Approaches at New Vaccines including Prevention of AIDS", CSH 1987, page 45). Like VV and cowpox virus, FPV has terminal sequences which are inverted repeats of each other and are covalently cross-linked together. A part of the terminal inverted repeat (TIR) sequence was digested with BamHI and cloned. The BamHI fragment was described as having a length of 6.3 kb. The poster describes the general layout of the sequences, which from left to right comprise a short unique region, a set of tandemly repeated sequences and then a long unique region containing three possible open reading frames. The summary notes that this FPV terminal fragment shares the general pattern of nucleotide sequence with the terminal fragments of VV and cowpoxvirus, but also notes some marked differences. The sequence of part of the VV TIR, namely from approximately nucleotides 6800 to 9000 from the left-hand end, VV is reported by S. Venkatesan et al., J. Virology 44, 637-646 (1982).

Large differences between the DNA sequence of VV and FPV are to be expected, since the FPV genome is estimated to be at least one third longer than that of VV. It seems likely, from the present knowledge as cited above, that many of the differences between the genomes of PFV and VV will be nearer to the termini than to the centre of the genome. Thus, information about the terminal region of VV is of limited interest in relation to FPV.

It has been a problem to locate a well-defined non-essential region other than the TK gene, in fowlpox virus. Very recently (20th April 1989), in PCT Application Publication No. WO89/03429 (Health Research Inc.), FPV recombinants have been described, but the non-essential regions mentioned therein are not all well defined. The recombinants were generated merely by cleaving the FPV gene into fragments and trying the fragments with test constructs to see whether the foreign gene under test was expressed.

The VV HindIII-D fragment is one of the best characterised regions of the virus. The sequence of this 16060bp fragment has been determined, Niles et al., Virology 153, 96-112 (1986), and thirteen genes (D1-D13) have been identified.

Several temperature-sensitive mutants have been mapped to genes within HindIII-D and fine mapping has assigned these to D2, D3, D5, D6, D7, D11 and D13, Seto et al., Virology 160 110-119 (1987). These genes are therefore essential for virus replication. It is not known whether the remaining genes within the VV HindIII-D fragment are essential or non-essential for virus growth. Recent experiments have suggested (though not proved) that D8, which encodes a transmembrane protein, might be non-essential for propagation of vaccinia virus in tissue culture, Niles and Seto, Journal of Virology 62 3772-3778 (1988). In these experiments, a frameshift mutation was introduced into the carboxy-end of D8 which removed the carboxy-terminal 56 amino acids of D8. Virus containing this mutation had growth rates indistinguishable from those of wild type virus.

It was not predictable, however, whether the D8 gene would occur in fowlpox virus and if so whether it would be non-essential. Moreover, there was the problem of how to locate the D8 gene in the FPV genome which is relatively unmapped and is much larger than that of VV. Consequently, examination of the vaccinia Virus HindIII-D fragment did not indicate how to find further non-essential regions within FPV.

### Summary of the invention

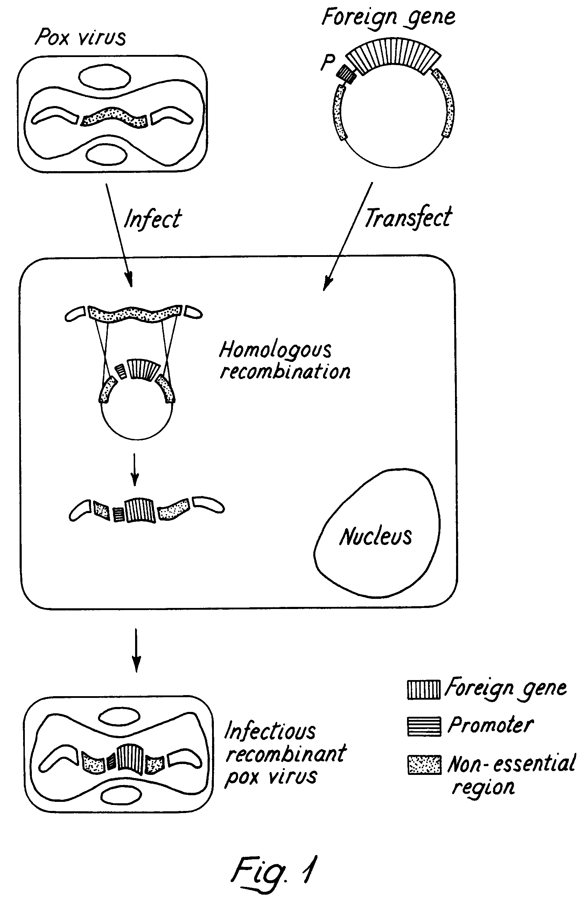

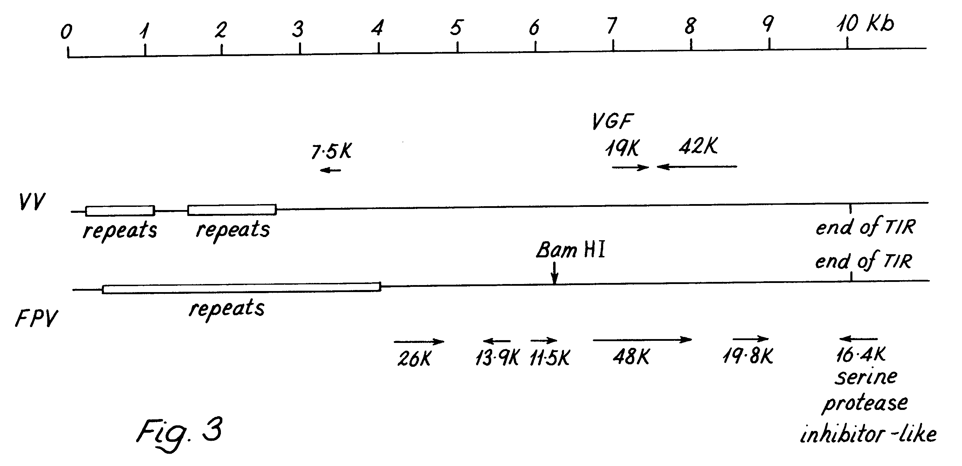

It has now been found that the three open-reading frames (ORFs) wholly within the above-mentioned BamHI fragment of the terminal inverted repeat (TIR) are non-essential regions which can serve as sites for homologous recombination events whereby a foreign gene can be introduced into FPV (refer to Figure 1). These ORFs are characterised in that they have lengths of approximately 670, 370 and 300 nucleotides and fall within the long unique sequence (LUS) of the TIR within the BamHI fragment. The BamHI fragment has been found to have a length of about 6.3kb and the long unique sequence begins at approximately nucleotide 4100, numbering from the left-hand end. (As to nucleotide numbering, please refer to the cautionary note immediately preceding the DNA sequence shown hereinafter). The lengths of the ORFs can also be expressed in terms of the approximate molecular weights of the polypeptides which they are predicted to encode, namely 26.0, 13.9 and 11.5 kilodaltons. This nomenclature will be used hereinafter for identification purposes. The 26.0 and 11.5 genes are coded for on the same strand and in that order when reading the fragment left to right from the 5' end towards the BamHI site, the 11.5 gene having its 3' near the BamHI site (see Fig. 3). The 13.9 gene is encoded on the complementary strand, reading right to left, but within a region distinct from and lying in between that coding for the 26.0. and 11.5 genes on the first strand.

Extrapolation from this and other findings leads to the reasonable suppositions that the whole of the long unique sequence of the TIR is a non-essential region, including sequence lying to the immediate right-hand end of the BamHI site, i.e. within the 11.2kb fragment sequenced by F.M. Tomley et al., loc. cit. (1988). The TIR has a length of about 10 kb, i.e. 10 - 6.3 = 3.7 kb lies within the 11.2 kb fragment. Thus, the long unique sequence extends to the right from about nucleotide 4100 over the remaining lengths of TIR, of about 6 kb.

Accordingly, in an important aspect the present invention provides a recombination vector for use in producing a recombination fowl pox virus (FPV) by homologous DNA recombination, said recombination vector comprising a cloning vector containing a non-essential region (NER) DNA-sequence lying within (and therefore consisting of at least part of) the long unique sequence (LUS) of the terminal inverted repeat (TIR) of FPV, said NER being interrupted by a foreign gene or genes and by a sequence or sequences for regulating the expression of each foreign gene present.
Preferably the NER is within part of the long unique sequence (LUS) lying within the 6.3 kb BamHI fragment. In a more particular aspect, the NER consists of at least a homologously recombinable length of an open reading frame (ORF) having a length of approximately 670, 370 or 300 nucleotides and falling wholly within that part of the LUS within the BamHI fragment of the TIR. However, we have also found that some isolates of the same strain of FPV do not have a lengthy sequence of over 200 bp of DNA spanning the BamHI site, which is an important pointer to the non-essentiality of the sequence spanning that site. It is therefore inappropriate to use the terminology "BamHI fragment" in a formal definition applicable to FPV generally. Accordingly, these ORFs are better defined as lying within the first 2.2 kb of the LUS after the tandem repeats, that is to say within 2.2 kb of its external end. NERs within this 2.2 kb are preferred. The above preferences for location of NERs apply also to the flanking sequences contained in the recombination vector and this invention, i.e. all the homologously recombinable sequence which flanks the foreign gene(s) and their regulatory sequence(s).

We have also found certain other non-essential regions of fowlpox virus. Whereas those described above are within part of the terminal inverted repeat, these further regions are in a more central part of the FPV DNA molecule, namely within or adjacent to one end of a 6.3 kb fragment of an EcoRI library of FPV, the genes of which show significant degrees of homology with certain genes of the vaccinia virus (VV) HindIII-D fragment.

This second aspect of the present invention has its origins in a random cloning of parts of the FPV genome, sequencing of the random clones and a search in all six reading frames for possible open reading frames which might predict genes. The sequences thus generated were compared with vaccinia virus (VV) protein sequences. It so happened that degrees of homology were noted to some of the VV proteins D1-D13, including approximate matches to VV D1, D5, D9, D10 and D13. It was therefore decided provisionally to adopt the "D" terminology for the FPV genes which showed some degree of homology with the VV genes. An M13 clone of FPV matching VV D9 was used as a probe to identify clones within an EcoRI library of FPV. One such clone, designated pMB379, containing a 6.3kb insert, was sequenced. This clone contained part of the D9 gene and all of D10, D11, D12 and D13. (The last four genes showed 40%, 62%, 53% and 55% identity of amino acids encoded to their VV homologues). In order to clone D7 and D8 genes, a DraI library of FPV DNA cloned in the plasmid pUC13 Sma1 was constructed. Colonies were probed with a prime-cut probe derived from an M13 clone designated M379-27S (which lies within the FPV D9 gene) and one positive colony, containing a plasmid designated pMB389, was studied further. pMB389 was found to contain the "carboxy"-end of the D6 gene, entire D7, D9, D10 genes and the "carboxy"-end of the D11 gene. (D11 runs in the reverse direction to D10, so the carboxy end is that nearer to D10. The "carboxy" end is that which is the 3′- end of the coding strand, corresponding to the carboxy end of the predicted polypeptide). There was no identifiable homologue of the VV D8 gene, although another gene was present between D7 and D9. This has been termed "D8" as a matter of convenience. In a particularly unexpected finding, the copy of D9 present in both pMB379 and pMB389 possessed frameshift mutations when compared with its VV counterpart, thus making it unlikely that D9 is expressed in FPV.

It has now been found that the D8 and D9 genes of FPV are non-essential genes which can be used for the insertion of foreign DNA. Since the intergenic regions can also be considered non-essential, it follows that in principle the whole of the region following the carboxy-end of D7 and up to the carboxy end of the D9 gene is a non-essential region (NER). (The genes D7, D8, D9 and D10 all run in the same direction). However, as will be explained hereinafter, the NER does not run right up to the carboxy-end of the D9 gene, but stops short thereof in order to avoid possible interference with the D10 gene, which overlaps with the D9, and the promoter of the D10 gene. This NER will be referred to hereinafter for brevity as the D8-D9 NER.

Accordingly, the present invention further provides recombination vector as set forth above but wherein the NER is within such a carboxy end-D7 to near carboxy end-D9 region NER (but excepting the D10 gene and D10 promoter region).

In a second important aspect the invention provides a recombinant vector, e.g. a plasmid, containing a NER sequence (TIR or D8-D9) as defined above. Such a vector is useful for preparing the above-defined recombination vector.

Thirdly, the invention includes a DNA molecule which consists substantially or essentially of a NER sequence as defined above.

Fourthly, the invention covers a host, appropriate to the vector, harbouring any of the vectors of the invention, i.e. the recombination vector or any intermediate vector which contains the novel NER sequence.

Fifthly, the invention embraces a recombinant FPV produced by homolgous recombination of a parent FPV with the insert DNA of a recombination vector defined above.

Sixthly, the invention includes the above-identified recombinant FPV and an in vitro culture of animal, especially chicken, cells infected with the above-identified recombinant FPV, for use in vaccinating a responsive animal, especially a chicken, with the recombinant FPV.

In a more particular aspect the recombination vector of the invention comprises in order:
(1) a first homologously recombinable flanking sequence,
(2) a sequence within a first portion of the non-essential region,
(3) promoter DNA,
(4) a foreign gene transcribably downstream of the promoter (whereby when the fowlpox virus RNA polymerase binds to the promoter it will transcribe the foreign gene into mRNA),
(5) a sequence within a second portion of the same non-essential region, the first and second sequences being in the same relative orientation as are the first and second portions of the non-essential region within the viral genome, and
(6) a second homologously recombinable flanking sequence.

### Brief description of the drawings

Fig. 1 is a schematic illustration of a homologous recombination process of the invention;
Fig. 2 is a map of the BamHI terminal fragment of FPV DNA showing the location therein of the beginning of the long unique sequence of the TIR;
Fig. 3 is a DNA map of the FPV TIR showing three non-essential region sequences useful in the present invention and other ORFs, together with a map of the VV TIR showing the different ORFs found in VV;
Fig. 4 to 7 illustrate schematically various general methods of construction of recombination plasmids in accordance with the invention; and
Fig. 8 illustrates schematically the construction of specific recombination plasmids of the invention containing non-essential region sequence of FPV into which a foreign gene is inserted.
Figure 9 is a map of the genes D6 to D13 and an adjacent gene A2 showing the genes cloned in plasmids pMB379 and 389 and important restriction sites in these plasmids;
   and
Figure 10 illustrates schematically the construction of specific recombination plasmids of the invention containing sequence within the D8, D9 and D10 genes (D8, D9 according to the invention; D10 comparative) into which a foreign gene is inserted.

### Description of the preferred embodiments

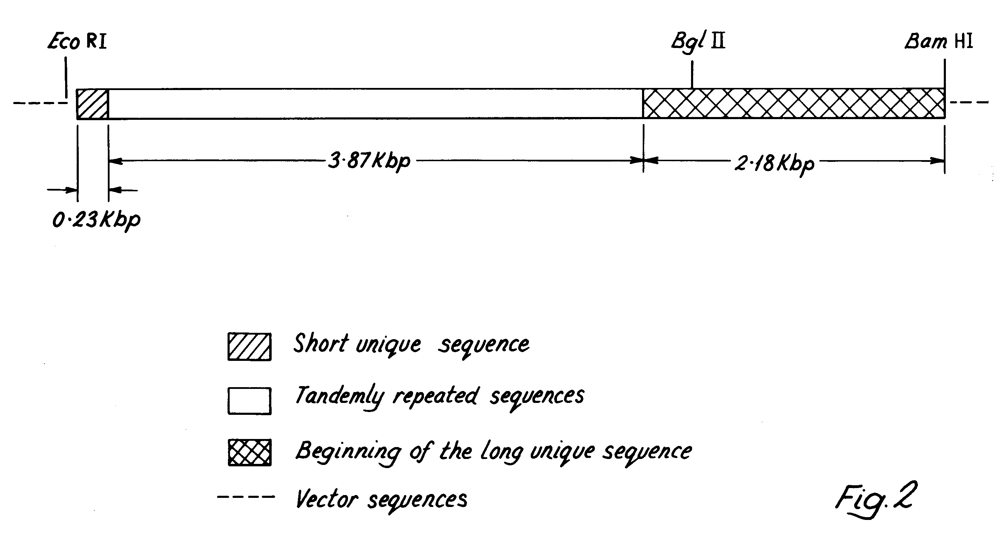

Referring first to the terminal inverted repeat (TIR) NERs and to Figure 2, the BamHI fragment of the TIR of FPV has a length of about 6.3 kb. It contains a short unique sequence of length 0.23 kb, followed by a region of approximate length 3.87 kb which contains tandemly repeated sequences and then a long unique sequence of length 2.18 kb.

The approximate location of the genes providing the TIR NER DNA for use in the invention within the BamHI fragment is shown in the map of Figure 3. The arrows show that the 13.9 gene is on a complementary strand. From this map and from sequence information it will be seen that these genes have no known near counterpart in VV. The BamHI site is believed to be part of a further non-essential region for the reasons explained above. A total of five ORFs have been found in the long unique sequence of the TIR. Those which appear to encode polypeptides by virtue of their distinctive FPV codon bias occur as follows :-

| Location (Nucleotides numbered from left-hand end) | No. nucleotides | No. amino acids encoded | M.w. of polypeptide | Unique site |
|---|---|---|---|---|
| 4125 - 4790 | 666 | 222 | 26.0 kd | Bgl II |
| 5261 - 5629C | 369 | 123 | 13.9 kd | Nco I |
| 5883 - 6185 | 303 | 101 | 11.5 kd | Spe I |
| C = occurs on the strand complementary to that shown in the nucleotide sequence below. | | | | |

Besides the three ORFs designated 26.0, 13.9 and 11.5, there are two shorter ORFs on the complementary strand. Since, however, neither of these ORFs exhibit the required FPV codon bias it is unlikely that they code for genes.

Below is shown the DNA sequence of the BamHI fragment SEQUENCE No. 1), starting from the left-hand end and reading in the usual 5' to 3' direction, together with predicted polypeptides encoded. It should be borne in mind, however, that the sequencing of the tandemly repeated region has presented difficulties and that the number of nucleotides within this region might be subject to some revision. This is not a matter of any significance to the present invention, which is not concerned with the tandemly repeated region. It follows that the long unique sequence does not necessarily start at about nucleotide 4110 in absolute numbering and the nucleotide numbers are therefore to be treated as merely guidance to the reader to enable him to identify the ORFs referred to and to be construed accordingly in the description and claims. SEQUENCE NO. 1 
1 ACCCTCATCTTACGATGAGTATTTATATAGTAAAAAAATGTATAAACAGTACCTTCCAA

61 AACCCTAATATACACATTCTTTACCCCTTAATTTGTTAAGGTGTAAAATACCCCCTATT

121 AAAATATATATTATTGTTTTAATAAAAAAACCATACGGTTTTACATAAAATAATACTAT

181 ATCTAATTTCCTTCCGGAAAATATTTATAAAGCTACCCAACGTTAGCGAAAAACTTTTT

241 TATCGAGAGCTCGAGTTATACAAAAAGTTTTTATCGAGATTTCGAAAAGCTTTTTTATCG

301 AGAGCTCGAGTTATACAAAAACTTTTTTATCGAGAGCTAGAGTTATAGAAAAAGTTTTTA

361 TCGAGATTTCGAAAAGCTTTTTTATCGAGCGCTCGAGTTATAGAAAAACTTTT.......

...... continuation of combinations of blocks of repeated

sequences..........

3721 .ATAGAAAAAGTTTTTATCGAGATTTCGAAAAGCTTTTTTATCGAGAGCTCGAGTTATAG

3781 AAAAACTTTTTTATCGAGAGCTCGAGTTATAGAAAAAGTTTTTATCGAGATTTCGAAAAG

3841 CTTTTTTATCGAGAGCTCGAGTTATAGAAAAACTTTTTTATCGAGAGCTCGAGTTATAGA

3901 AAAAGTTTTTATCGAGATTTCGAAAAGCTTTTTTATCGAGAGCTCGAGTTATAGAAAAA

3961 GTTTTTTATCGAGAGCTCGAGTTATAGAAAAACTTTTTTATCGAGAGCTCGAGTTATAGA

4021 AAAACTTTTTTATCGAGAGCTCGAGTTATAGAAAAACTTTTTTATCGAGATTTCGAAAAG

↓(4101) M I V E K I
4081 CTTTTTTTTATCGAGAGCTCGAGAAGTTAAATCGAGACGCCGATATGATCGTAGAGAAAA

A A W L L Y P L C L L R C F L C N S V R
4141 TAGCGGCGTGGTTATTGTATCCGCTATGCCTTCTACGATGTTTCCTCTGTAACTCGGTAA

P A T C K C V H C L L Y P F E V C C E C
4201 GGCCCGCCACTTGCAAATGCGTCCACTGTCTCTTGTATCCCTTCGAAGTATGTTGCGAAT

M S E T L D S L E H S C C Y C C V L P L
4261 GCATGAGCGAGACGTTAGACTCTCTGGAACACAGTTGTTGTTACTGTTGCGTGCTTCCTC

L I I R E F W R R V I L P T L K A T C D
4321 TATTGATTATCAGAGAGTTCTGGAGACGCGTGATATTACCTACTCTAAAAGCGACTTGCG

C I R L P C V L T R R F C K R T I C P L
4381 ACTGTATTAGGCTACCGTGCGTTCTCACCAGAAGATTCTGTAAAAGAACCATCTGCCCGT

A K S W C R C F C C P C E V F L R C L L
4441 TAGCTAAATCTTGGTGTCGTTGTTTCTGTTGCCCTTGCGAGGTTTTCTTGAGGTGTCTCC

F P C M M L R R M H R G R L T G V R E P
4501 TCTTTCCTTGCATGATGTTGAGAAGAATGCATAGAGGCAGACTGACCGGAGTAAGAGAAC
 
G A F R D S R D P A R R G T W V N D W C
4561 CGGGAGCGTTTAGAGATTCAAGAGATCCCGCCAGACGGGGAACCTGGGTCAACGACTGGT

Bgl II
E D L C V W I W S P C C Y V K R C I R T
4621 GCGAAGATCTCTGCGTATGGATATGGTCTCCGTGTTGTTACGTCAAGAGATGTATTCGCA
------

M C D T F T K K I F Y W F I A P A G S P
4681 CGATGTGCGACACCTTCACTAAAAAAATTTTCTACTGGTTCATCGCCCCCGCAGGATCGC

R M P E E P S P L S R K V F S S *
4741 CGAGAATGCCCGAGGAACCTTCTCCGCTATCGAGAAAGGTCTTTTCGTCGTGAAGCATCG

4801 CGACGTGTAGATGAAAGAGATTGGACGCCAAAAGGTATACGCTCGTATTATTACGAATCG

4861 CGCTCGTCGTCGTGCCTCGAAGATGATAAAGAGAACGCGAGTAAGGAAATACAAGATAAA

4921 AACAAACTGTCGATTCTACGTAATACGATTTCTAAAACGGCGTCTACCGCAATCTTTTTC

4981 ATCAGAGTAAGGGATAGGTTAAGATTCTTAAGGACCTTTATCAAAACTATCGGAGAAAAA

5041 CTTTACGCTAAAGCCGCTCTCATGCTCTTTCACAGACTGGTGACCTATAGGCTTCCTATC

5101 GTTAGGGATATAGTCCCGTTTTATTACGCGAGGAAAAATTTTTGGAGGTTCGCTTTTCTA

5161 TATCTCATGAAGAAAGTCGCTGTTCGTCCTACTAAAAACTAATGCTAAAAAAAAATCTAT

5221 CGTTAATAAATTAAAAGTTATCGGGTTTTGAATATTATTAATCTATGCATACGAAACCGT
* D I C V F G Y

5281 ACGTCGCGTTACACGGGGAGAATCCGAACGTTTTCCCGTCCGTATAGGCACACTTACCCA
T A N C P S F G F T K G D T Y A C K G V

5341 CTTCTGGTTTCTTGTCATCCCTTATCCACAAACTCTGCCCCATACCGAATTTGCTTATGA
E P K K D D R I W L S Q G M G F K S I F

Nco I
5401 ATTTCATGTCTTTTTCATTGTCTATTAGACTACGTCTAGCTAACGTACCCATGGGAAACG
------
K M D K E N D I L S R R A L T G M P F T

5461 TTATCAAACACGTAGTCGACGCTTCGCTGTAAGTTTTTTTGTGATGCATTTTATCTAATC
I L C T T S A E S Y T K K H H M K D L R

5521 GGTTTACCCAGATACACTTATTGTTGTAAGAGGTGTACGGTCCTCTGCAATTTAGAGAGT
N V W I C K N N Y S T Y P G R C N L S N

5581 TAGCGCAATCCAACATTAACAACATCGCTATCAAGATTAATGATTTCATTTTGATACACG
A C D L M L L M A I L I L S K M

5641 GATCAATTTTAATAGTCTATAGAGATATACCCCACCAATAGTTACGCGATTAATTTTCAA
 
5701 TTATACATAATAAAAATCTAGAGGAGTTGCCATGTGATTCTCTGTCGACGACGTTACCTC

5761 GTTAGTTTTATTAATTCTTCTTTAAGTTCTGACGACGGTTATATACCAAAACAGCTTTTA

5821 CTTTTCGTAAGACGATAATTACGTTATAAAAGATTAAGAAATTTTATTTTTACTAAAGTA

M N N D T I F T L F Y C K N K K Y V R G
5881 TCATGAATAATGACACGATATTTACTTTGTTTTATTGTAAAAATAAAAAATATGTTCGTG

E G G R R R G K T G I L L F H P I N H R
5941 GGGAGGGGGGAAGGAGGAGAGGGAAGACGGGTATCCTTTTATTCCATCCAATAAACCACC

Spe I
V I G T S A H Q C Y K T R R I G F K L Y
6001 GTGTTATAGGAACTAGTGCACATCAGTGTTATAAAACACGAAGAATCGGTTTCAAACTCT
------

A V A P R H V S T I R C G R S H S A H R
6061 ACGCAGTTGCCCCTCGTCACGTATCCACTATCCGCTGTGGAGATCCCACTCGGCTCACC

V D K F S F S F Q K V D F H C I A G S G
6121 GAGTAGATAAGTTCTCCTTCTCCTTCCAAAAAGTAGATTTTCACTGTATCGCCGGCAGCG

A *
6181 GCGCATAGGTATCTGGCCCTACAGAATTCCATATTCCTGATAGCTCGCGCGACGAAGCCT

6241 CCGTCGCCCGTGTGATCATTTTCATAATTCAAGGGGATCC (6280)
It will be appreciated that the sequence shown above is not definitive of FPV generically, since there will inevitably be small variations between different types or strains of FPV, some of which might take the form of deletions or insertions, and any variant NER compatible with the desired homologous recombination is usable herein. In particular, DNA having a NER sequence based on ORFs having a sequence showing at least, say, 90% homology with the sequence set forth above is a preferred feature of the invention.

Beyond the BamHI site (GGATCC) the nucleotide sequence continues as per F.M. Tomley et al. loc. cit. (1988) at page 1029, nucleotide 6275 shown above being the first of the BamHI recognition sequence and No. 1 in Tomley et al.'s numbering. While there are no ORFs spanning the BamHI site, the sequence extending at least as far as the first 160 bp into the (11.2 kb fragment of Tomley et al. is demonstrable non-essential, while further sequence thereafter is likely to be.

The NERs of the invention can be used to insert foreign genes into FPV. It is desirable that the gene is stably incorporated so that the resultant recombinant FPV can be repeatedly cultured in tissue culture on a large scale. Evidence to date is that the 13.9 gene is less stable than the other two.

The foreign gene can be inserted at any location within the NER sequence of the recombination plasmid. Preferably it is not so excessively near one end or the other as not to provide a site long enough for homologous recombination with the corresponding NER of the wild type FPV. However, it is possible to allow homologous recombination to occur outside the NER by providing, in the recombination vector, flanking sequence lying beyond either or both ends of the NER. It is probably not usually beneficial to use flanking sequences which are heavily tandemly repeated, since this will increase the chances of unwanted recombination events. Thus, it is suggested that flanking sequences running extensively beyond the external end of the LUS into the tandem repeat region are best avoided.

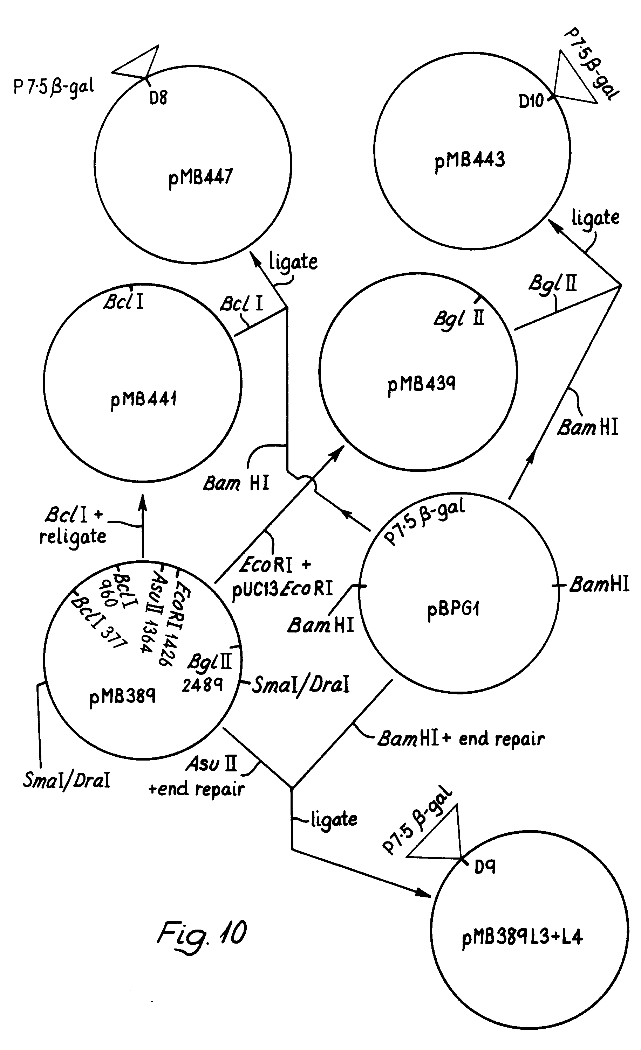

Referring now to the D8-D9 NER and to Fig. 9 of the drawings, the plasmids pMB379 and pMB389 have FPV DNA inserts which overlap in the D9 and D10 gene region. By sequencing these inserts a long nucleotide sequence has been obtained which runs from the 3'-end of D6 through to D13 and beyond that to the adjacent A2 gene.

In the sequence shown below (SEQUENCE No. 2), those parts which are not relevant to the present invention have been truncated. The restriction sites Dral (TTTAAA), EcoRI (GAATTC), BclI (TGATCA), AsuII (TTCGAA) and BglII (AGATCT) used in the cloning and construction of plasmids for testing out non-essential regions are underlined. The amino acid sequences of predicted genes are given in the single letter code. The D9 gene, being considered to be unexpressed in FPV, is not given an amino acid sequence, but is considered to run from nucleotides 1298 to 1984. Start and stop codons have been underlined. The sequence shown encompasses the carboxy end of the D7 gene and the carboxy end of the D11 gene. (Since the D11 gene has an open-reading frame running in the reverse direction to the others, its near-D10 end is that encoding the carboxy terminus of the putative D11 protein). The sequence shows the DraI site in the D11 gene (see also Figure 9).

It will be seen that the D10 gene starts within the end of the D9 gene, being within a different reading frame. The D10 promoter is therefore also within the D9 gene. The non-essential region is therefore defined as extending up to the beginning of the D10 promoter region, which is estimated at approximately 35 bases before the ATG start codon for D10, i.e. at around nucleotide 1936.

SEQUENCE NO. 2

D7 (carboxy-terminus)

A Q S G M E S S F V F L G N I I E K *
1 AGCCCAATCTGGAATGGAATCATCTTTTGTTTTCTTAGGTAACATAATTGAAAAATAATA

61 CTTTAATAAAGATTAATTTACATAATACCTACATCATGTAACGTATAGCTTATTAAACAT

121 GGATAGCATTTAGTAATATTTGTAAAATCAAAAATAACTATAAATATTTTAATAGTATAT

181 TGAATTCTTTATCATGTAGAAAGATATTAGTTATACACACAATGATATGTAAAAATAGTA

241 TTCGACATAAAGAATTGACGTTGATAATATTTCTCTGCTTTATATATCTATCAAAAACTT

301 TTGTAGAAGTAAAAGCACCTCCCTATGTACTCGTACCGGAAGTAGTGATATAAATCTTAC

361 TTGTTTTATTAAAGATGATCAGGGTGCTGGAGAAGATAAAGTAATTGTAGCGTGGCAACA

421 GGGTGACGGAGAAGTTACTCAAGGAATAAAAACAACTTGGGATACGACACGACAAGTGGG

D8 M L H I S K V S K E A E G S Y M C V
481 CCAAACCATGCTACATATATCCAAGGTATCTAAAGAAGCAGAAGGTTCATATATGTGTGT

V W I N G D A D Y K K M N L G V F T V S
541 TGTATGGATAAACGGCGACGCTGATTATAAAAAAATGAATCTAGGAGTGTTTACTGTTTC

K K T Y M H N E V S I T P R R S R V D M
601 AAAAAAAACCTACATGCATAACGAAGTAAGTATAACCCCTCGAAGATCGAGAGTAGATAT

S D G R P L K I E C A F S T R R I Y G R
661 GTCGGATGGTCGGCCGCTAAAAATTGAATGTGCTTTTAGTACCCGACGAATATATGGTAG

S Q V N I K W W K I D G I T R K W E Q Q
721 AAGCCAAGTCAACATAAAATGGTGGAAGATTGACGGTATTACACGAAAATGGGAACAACA

T S G V N L L L H T Y G G I G S L S I P
781 GACTTCGGGAGTTAACTTACTGTTACATACTTATGGAGGGATAGGATCGCTAAGTATCCC

N P T T G E S T G K Y M C V V T C G D I
841 AAATCCTACAACAGGAGAATCAACAGGTAAGTATATGTGCGTAGTTACCTGTGGAGATAT

G N V G F R L V K S L S P L S D T E S D
901 TGGAAATGTTGGATTTAGACTGGTAAAATCATTATCTCCTTTATCAGATACAGAATCTGA

H S Y T S E E G S H F M E R C K V K K S
961 TCATAGTTATACTTCAGAGGAAGGATCACATTTTATGGAAAGATGCAAAGTCAAAAAAAG

P Y G G W I V E *
1021 TCCGTACGGTGGATGGATAGTAGAATAGATCATCTCAGGGACGCGGTATATATTGCTATA

1081 AAGTAAAATAATTTCTCAATACATTTTTCTACTTTAACGATATAATCGTTAACTTATTAG

1141 TTTAATTATATCATATATTCCTTTATATCTTAATTAAAAAAATATATTCATAAATGGGAT

1201 ACACTAGTATTTTTTTATATAGTTTACGATATGCTATTTCACTATATATAGTTTATCAAA

1261 CATAAGTGAAAAATAAACAACTATAAAAACTAAGATCATGTTCGATATATCCAGAGAACA
1321 ACAAAATATATTAGAAAAAAATAAAGATTGTGTTATTACTTTCGAAACAAATAAAGAATA
1381 ACTATAGAAAACACTAACATAAAAGATATACTTAGCGATAGACGAATTCACATATCGCGT
1441 TATGTATTACATCAGATAATATACCTATAATCGGTATAAGAAGAACGTCTTTTATGTATC
1501 AATCGGTTATATCAAAAAGAAGAAGCTTTTCAGAAATATTAGCCGTCGATATAAACCATC
1561 TAAAGTATATGTATAATAACGAAATTAAAGAAATATCATTAGATCAATAGTACCATTCAC
1621 ATATAGCGGATTCAACAACTTTGAAGAGTTAGTATTATTAGGAGGAAGAGTTAAAAATAA
1681 AGAATCAATATATCAATGCCTGAGTAGAGAACTATCGGAAGAAAGCGATGGAATACTTAC
1741 TATAAAAACATTTGGTAATAAAATATTAAAACTTACCATAGAAGATAAGATACTTCGTAG
1801 AACATTTTATGGGTATTGTATAGTGTGTTTTATAGACCAACTCTATTCAGAAATCATTAA
1861 ACCTTTATATAACATAGAAATCAAAGAATTAGGATCATTATTTGATCGATCAAGTAATGA
D10 M G E
1921 AAAATACGAATACTTGCATTTTATTTATAATACCTTATTAACATATAAATATGGGGGAGT
Y Y K N K L L L R P S V Y S D N I Q K I
1981 ATTATAAAAATAAATTGCTCCTTAGGCCGTCTGTATATTCTGATAATATACAAAAAATCA
K L V A Y E Y G K L H A K Y P L S V I G
2041 AACTGGTAGCATATGAATATGGCAAACTACATGCTAAATATCCACTATCCGTAATAGGTA
I M K T I D D K F V L C H R Y N S F L F
2101 TAATGAAAACAATAGATGACAAGTTTGTTTTATGCCATAGATACAATAGTTTCTTGTTTT
S E I A F T K D K R R K I R L F K K Y S
2161 CAGAAATAGCATTTACAAAAGATAAACGACGGAAAATAAGACTCTTTAAGAAATATTCAA
K Y M S N I E R D I L S Y K L S L P N N
2221 AGTATATGAGTAACATCGAACGTGATATATTAAGTTATAAGCTATCGCTTCCTAATAACT
Y N T N H I D I I F P G G K I K D L E S
2281 ATAATACAAACCATATAGATATAATCTTCCCAGGTGGTAAAATAAAAGACTTGGAAAGTA
I T N C L V R E I K E E L N I D S S Y L
2341 TAACTAATTGTCTAGTAAGAGAAATAAAAGAAGAATTAAATATCGATTCCTCTTATCTCG
A I C K N C F V Y G S I Y D R L I D K D
2401 CTATCTGTAAGAACTGTTTTGTATACGGTTCTATATACGACAGATTGATAGATAAAGATT
F E V I A L Y V E T D L T S R Q I L N R
2461 TTGAAGTTATAGCCCTTTACGTAGAAACAGATCTTACGAGTAGACAAATATTGAATAGAT

F I P N R E I K G I S F I D A R D I N K
2521 TCATCCCTAATAGAGAAATTAAAGGAATATCATTTATAGACGCAAGAGATATTAACAAAG

D Y L Y T N V I K Y I I N A V R T S A S
2581 ATTATTTGTATACTAATGTAATTAAATATATAATAAACGCTGTAAGAACATCCGCTAGCA

N S *
2641 ATAGTTAACATCTATCTAACTGTTAATATATAAATTAATTT....
D11 * R V T L I Y I L K
(carboxy-terminus)

2881 ......TAATTTTTTAAACCC......
L K K F G

There are some preliminary indications that the insertion of a foreign gene in the D10 gene is unstable. Accordingly the D10 gene is not a NER for the purposes of the invention.

Referring now to all the NERs of this invention, the recombination vector can contain a reasonable length of homologously recombinable flanking sequence to each side of the NER, preferably at least 100 nucleotides, more preferably at least 250 nucleotides and still more preferably at least 500 nucleotides. On the other hand, in the interests of not making the insert of the recombination vector too large, it is often desirable to limit such flanking sequence outside the NER to, say, not more than 1000 bp.

Incidentally, precise homology of the flanking sequence and the NER is not necessarily required. Remarks to that effect in WO 89/03429 apply here also.

In preparing recombinant FPV of the invention, the first requirement is for a promoter. The well-known vaccinia virus P7.5 promoter the H6 promoter (see the cited PCT Application Publication No. WO 89/03429) or the P11 promoter, can be used, as these are accepted by the FPV as compatible. Preferably, however, a FPV promoter is used. Certain promoters have been described in the prior PCT patent application Publication No. WO 89/03879 (National Research Development Corporation), the contents of which are herein incorporated by reference. The "4b" and "13.2K" promoters therein are especially preferred.

The invention has particular relevance to poultry, especially chickens. For this purpose, any foreign gene relevant to improving the condition of poultry could be inserted into the fowlpox virus. Preferably the gene will be one appropriate to an in vivo sub-unit vaccine, for example one or more genes selected from Infectious Bronchitis Virus (IBV), Infectious Bursal Disease virus, Newcastle Disease Virus (NDV), Marek's disease virus, infectious laryngotracheitis virus and genes encoding antigenic proteins of Eimeria species. Particular genes of interest are the spike genes of IBV and the HN and F genes of NDV as described in PCT Patent Application Publication No. WO 86/05806 and European Patent Application Publication No. 227414A (both National Research Development Corporation). In order for the foreign gene to be correctly translated in vivo it is necessary for the foreign gene to have its own ATG start codon inserted in the region just following the promoter.

More than one foreign gene can be expressed in a single poxvirus. It is possible to arrange for mRNA for two foreign genes to be transcribed in different directions when inserted in a single non-essential region. Such a "back to back" construction is shown in Example 7. Also, individual foreign genes can be inserted in individual NERs described herein. While fusion protein genes can, of course, be made, it is ordinarily preferable that each gene be under control of a separate promoter.

The promoter and foreign gene then have to be inserted into the non-essential region (NER) of the FPV genome. The procedure of homologous recombination, illustrated by Figure 1 of the drawings, provides a way of doing so. A fragment of genomic DNA containing the NER is sub-cloned in a cloning vector. A construct is then made, in the cloning vector, comprising part of the NER followed by the promoter followed by the foreign gene, followed by a further part of the NER in the same orientation as the first part. This construct, in an appropriate vector, forms the recombination vector which is used to transfect the FPV, e.g. by the calcium phosphate method, whereby recombination occurs between the NER sequences in the recombination vector and the NER sequences in the FPV. The FPV then automatically re-packages this altered genome and the thus altered FPV (recombinant FPV) is part of this invention.

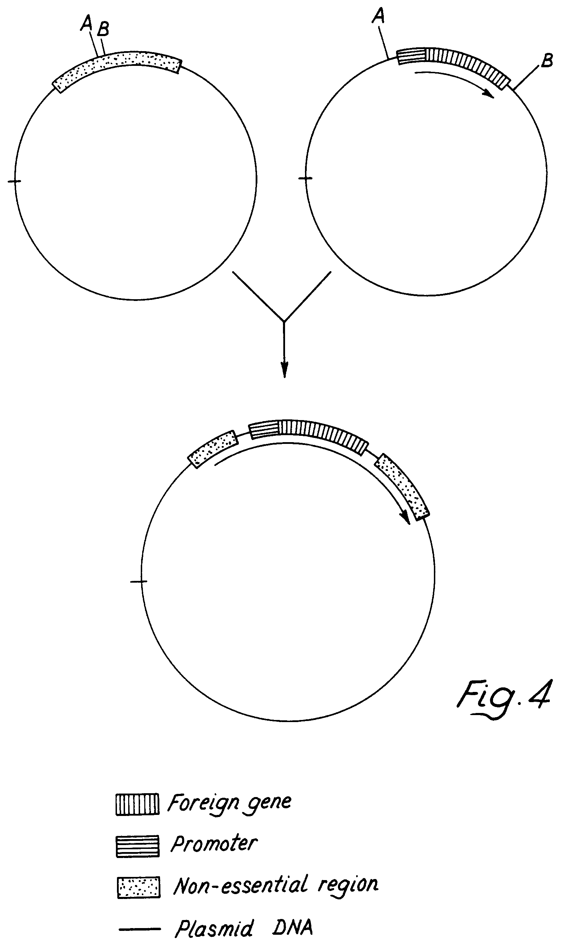

The above-described recombination vector can be prepared in many different ways, the requisite elements being introduced in any order. Referring to Figure 4, a non-essential region defined above for use in the invention possessing (say) two restriction sites A, B is inserted in an appropriate vector, which by way of illustration only will be described as a plasmid. In another plasmid having the same (or ligatably compatible) restriction sites A, B, a construct is made of promoter sequence followed by the foreign gene sequence. It is of course essential that this construct is made so that the mRNA transcription will begin at or before the start codon of the foreign gene. Since it is time-consuming to determine precisely where the mRNA transcription start is effected by any particular promoter, it is convenient simply to insert, say, 100 or more preferably 150 base pairs of promoter DNA immediately preceding the FPV gene which it normally promotes, to ensure good working of the promoter.

The restriction sites A, B are located in the plasmid DNA flanking the FPV promoter DNA and the foreign gene. Of course, A can be within the promoter DNA if it falls within a non-functional portion thereof. While A and B can be two different restriction sites, they can, of course, be one and the same. They can be sticky- or blunt-ended sites and can be prepared artificially, e.g. by filling in with additional nucleotides or chewing back, in ways well known in the recombinant DNA field. Conveniently A and B are or are converted into a single blunt-ended site (C) and then allowed to ligate into a single blunt-ended site (C) within the NER. Care will have to be taken, of course, to select sites which are unique in the plasmid DNA to prevent ligation of other sequences of DNA from occurring. In the Example herein, unique sites for SpeI, NcoI and BglII in the NERs have been used and have been filled in to provide a single blunt-ended site for insertion of the promoter-gene construct.

DNA from the two plasmids are ligated together in vitro and then transformed into the host, to produce the final recombination plasmid of the invention.

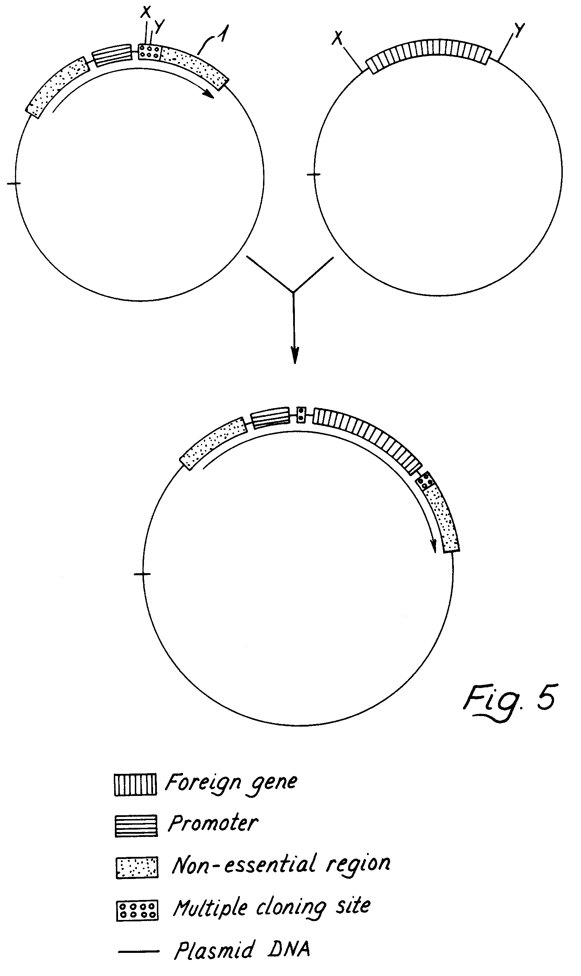

Figure 5 illustrates another method of preparing recombinant vectors. In this method one first prepares a construct comprising a first part of the NER, followed by the promoter, followed by a short sequence of nucleotides containing at least one cloning site for introduction of a foreign gene, followed by a second part of the NER which will almost inevitably be in the same orientation as the first part. Of course, virtually any length of DNA would provide a cloning site suitable in some way or other for introducing a foreign gene. Preferably these constructs contain a multiple cloning site, that is to say a length of DNA containing the sites of a variety of different restriction enzymes, for example at least ten. Such a construct then has versatility, since it will then be much easier to restrict DNA flanking almost any foreign gene at sites close to each end thereof and insert the foreign gene into the multiple cloning site illustrated in Figure 5. Only two sites X, Y have been shown, for simplicity and these can be filled in or chewed back, as desired, to give identical blunt-ended sites (Z, replacing X, Y). In the final recombination plasmids the promoter DNA will be separated from the foreign gene by a portion of the multiple cloning site, but this will not adversely affect the transcription of the mRNA in the final virus.

In either method of construction of NER is split by the promoter and foreign gene. It is, of course, not essential that it be split in a central region. Nor is it essential that the second part thereof constitute the entire balance or remainder of the NER. As explained above, so long as there exists within or at each end of the NER contains a long enough stretch of DNA for homologous recombination, it does not matter that a part of the NER might be excised somewhere in between or that additional (irrelevant) DNA be inserted in preparing the recombination plasmid, or even that the NER is only a few bp in length (but it should not normally be less than about 20 bp long or there would be practical difficulties in inserting the foreign DNA).

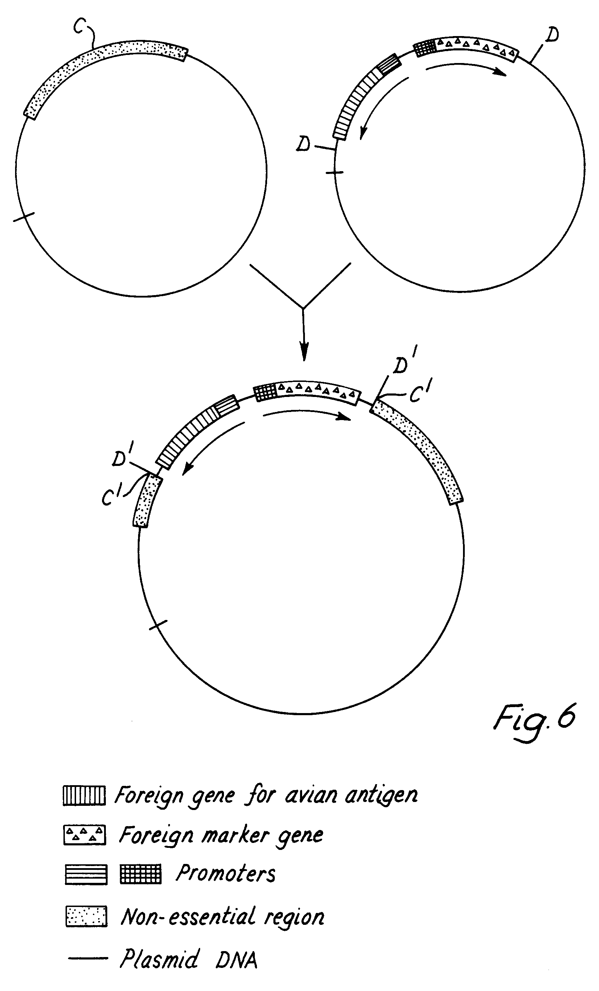

Figure 6 illustrates another method of preparing a recombination plasmid. The NER plasmid is restricted as before (a blunt-ended site or sticky ended site filled in to become blunt-ended, C, is illustrated arbitrarily for this Figure). The other plasmid contains a marker gene with its own promoter and a foreign gene with its promoter in opposed orientations. Different promoters should be used to avoid the possibility of an incorrect recombination. When these plasmids are ligated together the marker is linked to the foreign gene. The presence of the marker in the recombinant FPV is therefore likely to indicate a successful incorporation of the foreign gene into FPV. Conveniently the marker is readily detectable by a colour, fluorescent or chemiluminescent reaction. The beta-galactosidase or lacZ gene can be inserted into the NER and recombinants detected by the blue plaques generated when the 5-bromo-4-chloro-3-indolyl-D- galactopyranoside (X-gal) substrate is present in the growth medium. This technique, using the lacZ gene, has been described in relation to VV by S. Chakrabarti et al., Molecular and Cellular Biology 5, 3403-3409 (1985).

Preferably the marker gene is also selectable (or it is replaced by a selectable gene or two separate genes, one marker and one selectable are included). An example of a selectable gene is the Ecogpt gene described in PCT Application WO 88/02022, already cited, and also by F.G. Falkner and B. Moss, J. Virology 62, 1849-1854 (1988).

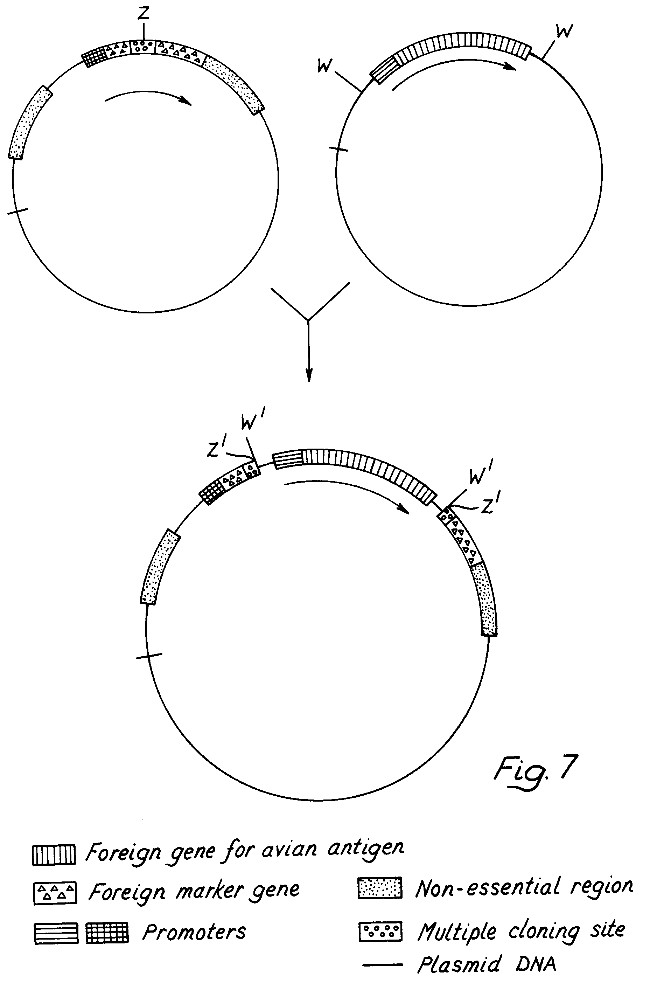

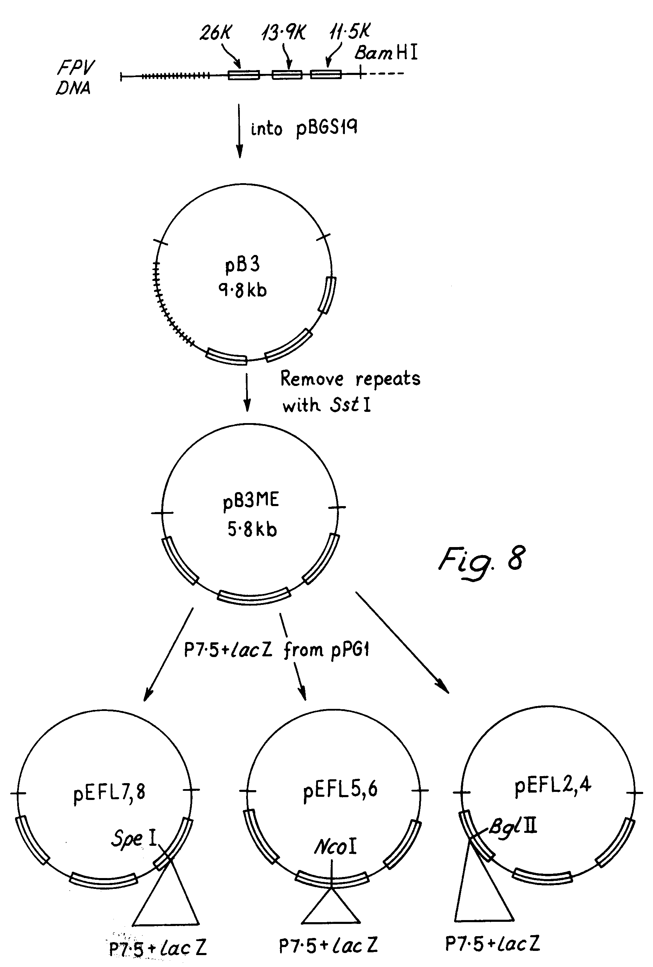

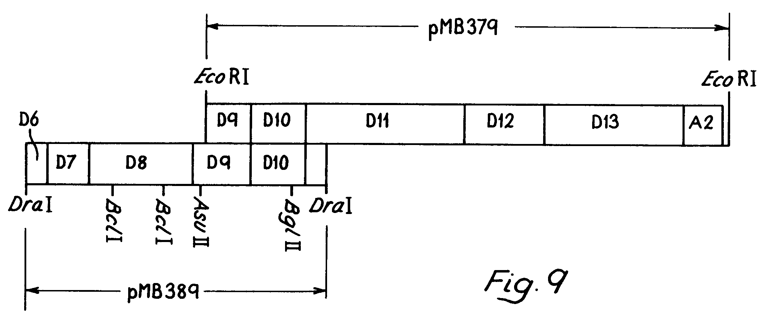

It is also useful when preparing a recombination plasmid to known that the foreign gene is likely to have been successfully inserted into the NER. One way of checking is to adopt the procedure shown in Figure 7. Again, blunt-ended sites (Z and W) are shown. In a first plasmid, containing the NER, a marker gene is inserted within the NER and optionally a multiple cloning site is inserted within the marker gene. The foreign gene with its promoter, contained in a separate plasmid, is then inserted into the marker gene by combining the two plasmids as shown. The foreign gene is inserted at any site within the marker gene which will interrupt or change its marker function, whereby the insertion can be recognised. For example, the E. coli. lacZ gene can be used as the marker gene. Only the alpha-fragment need be inserted since the remaining elements of the gene required for processing are provided by the bacterial host. It is provided with the E. coli. promoter for lacZ, the lacZ start codon and, preferably, a multiple cloning site within a few codons of the start, followed by sequence encoding the alpha fragment. When the alpha-fragment is interrupted by a foreign gene, conveniently insertable in the said multiple cloning site, the blue colonies given on a X-gal substrate are not ordinarily obtained. White colonies indicate a likely recombination plasmid.

The recombination vector could, on occasion, be further provided with a termination signal site to terminate transcription of mRNA, which might be useful for stabilising the mRNA when an early-acting promoter is used. This would be inserted downstream of the foreign gene. However, in many cases the ordinary termination signals of the infecting poxvirus or the signals for starting transcription of another, downstream, gene of the FPV will be adequate.

References herein to vectors other than FPV (or VV) mean any convenient prokaryotic or eukaryotic cloning vector appropriate for bulk production of the construct within a suitable host. Prokaryotic vectors will ordinarily be plasmids or phages. Suitable prokaryotic hosts include bacteria. Eukaryotic vectors such as those of fungi, yeasts and animal cells, e.g. SV40, can be employed if thought more convenient.

Although the recombination vector used will ordinarily be of double-stranded DNA, it is possible to use single-stranded DNA for the homologous recombination.

The recombination vector of the invention containing the NER, promoter and foreign gene then ahs to be "swapped over" for the "parent" FPV DNA. For this purpose, appropriate animal, preferably but not necessarily poultry, cells are infected with the parent FPV, and the recombination vector is then introduced into the cells. This process is carried out in vitro. It is best not to use wild type parent FPV for obvious reasons. FPV can readily be attenuated (mutated to make it less virulent), bu any conventional method of attenuation.

Many different methods are available for detecting or selecting the recombinant viruses, and have been described for VV in a review article of M. Mackett and G.L. Smith, J. General Virology 67, 2067-2082 (1986). Such methods are applicable in the present invention. One method is to enlarge the recombination plasmid to include along with the desired foreign gene an additional marker gene as described above (Figure 6). Alternatively, the insertion of the foreign gene can be detected by a hybridisation assay in which a labelled nucleotide sequence complementary to the foreign gene sequence is employed. Such assays are well known.

The FPV recombinants possessing the foreign gene are then grown in appropriate cells. The NER of the invention are non-essential in chicken embryo fibroblast cells. It would be necessary to carry out routine experiments to determine whether they are also usable for growth of FPV in other kinds of cell, e.g. chicken embryo skin cells, chicken fibroblasts, chicken embryo epithelial cells derived by conventional tissue culture methods, principally trypsinisation of tissues or the chorioallantoic membrane (CAM) of embryonated chicken or turkey eggs.

For administration to birds as a vaccine, the recombinant virus can be given to birds by any suitable method such as by aerosol, drinking water, oral, intramuscular injection or inoculation into the wing web. Ingredients such as skimmed milk or glycerol can be used to stabilise the virus. It is preferred to vaccinate chicks 1 day old by aerosol administration. A dose of from 10² to 10⁸ pfu, preferably 10⁴ to 10⁶ pfu, of the recombinant FPV per bird is recommended in general.

While the invention is intended primarily for the treatment of chickens, it is potentially of interest in relation to the vaccination of other animals which might safely be inoculated with FPV. It is even possible that it might be considered safe to infect humans with FPV after appropriate trials have taken place.

The following Examples illustrate the invention.

### EXAMPLE 1

This example relates to terminal inverted repeat NERs.

### Virus strain

The HP438 strain of fowlpox virus was obtained from Professors A. Mayr and H. Mahnel, Ludwig-Maximillians University, Munich. The HP438 strain has been obtained from the pathogenic HP1 strain by 438 passages in chick embryo fibroblasts (CEFs) in tissue culture. A. Mayr et al., Zentralblatt für Veterinärmedizin B13: 1-13 (1966). The HP 444 strain used to obtain DNA for cloning was derived by 6 further passages in CEF cells.

### Tissue culture medium

CEF cells were grown in 199 (Wellcome) medium, supplemented with Penicillin (200U/ml), Streptomycin (200µg/ml), Fungizone (2µg/ml) and 10% newborn calf serum.

### Cell Culture and Viral DNA purification

FPV strain HP444 was grown in primary chick embryo fibroblast (CEF) cultures and FPV DNA prepared as described by M.M. Binns et al., Nucleic Acids Research 15, 6563-6573 (1987).

### Identification of the terminal fragment

The genome of poxviruses is double-stranded DNA. As usual the two strands are held together by hydrogen-bonding. However, at the ends of the genome of poxviruses the two strands are covalently linked. Topologically this means that the DNA is a continuous strand. When the genome is cut into fragments with a j restriction enzyme, internal fragments are topologically different from terminal fragments and behave differently. On heating (to separate the two strands) the internal fragments completely separate and they reanneal slowly (hardly at all if one chills quickly). The terminal fragments, being covalently bonded, cannot separate very far spatially. On cooling they reanneal quickly by starting at the middle and "zipping up". This is called 'snap-back'. After heating and rapid cooling therefore, only the terminal fragments can be seen by gel electrophoresis. This method of identification of the terminal fragment, called "the snap back method," see L.C. Archard & M. Mackett, J. General Virol, 45, 61-63 (1979), was used here.

A BamHI restriction (10µg) DNA was extracted once with phenol, the aqueous phase was extracted with diethyl ether to remove phenol and the DNA in the aqueous phase was precipitated with ethanol. The DNA pellet was dried and resuspended in 20 µl of TE (10mM Tris HCl pH 7.5, 1mM, EDTA). The DNA solution (10µl) was then denatured by the addition of 40µl of 95% v/v deionized formamide in 50mM Tris HCl pH 7.5 and incubated at 60°C for 10 minutes. The sample tube was then rapidly cooled on wet ice containing NaCl for 10 minutes before analysing the DNA by agarose gel electrophoresis. The presumed terminal fragment renatured rapidly only in the native FPV DNA and was detected as an approximately 6.3kb band. There is only one BamHI - derived snap back fragment, as the BamHI site falls well within the TIRs.

### Cloning of the end fragment of FPV DNA

The terminal crosslinks of the BamHI fragment were digested with S1 nuclease; using 100µg of FPV DNA and 20 units S1 nuclease (Boehringer Mannheim) in 250µl of 30mM sodium acetate, pH 4.5; 0.3M NaCl; 1mM ZnS0₄; 5% (v/v) glycerol at 37°C for 20 minutes. To end-repair the DNA, it was then ethanol-precipitated and resuspended in 100 µl of ligation buffer (50mM Tris. HCl pH 7.5, 10mM magnesium chloride, 10mM DDT) containing 5 units of E. coli polymerase I Klenow fragment and 1 unit of T₄ DNA polymerase and 0.025 mM each of dATP, dCTP, dGTP, and dTTP. This reaction was left for 1 hour at room temperature. It was stopped by heating for 10 minutes at 70°C and 20 µl thereof was digested with 10 units of BamHI (Boehringer Mannheim) in accordance with the manufacturer's recommendations. 5µl of this DNA was ligated to 20 ng of BamHI/Sma I - cut plasmid pBGS19, B.G. Spratt et al., Gene 41, 337-342 (1986), to make a total volume of 20µl ligation buffer containing 2 units of T₄ DNA ligase and 1mM ATP, and incubated at 4°C overnight and transformed into E. coli. Screening for recombinants which contained the end fragment was carried out with a radiolabelled FPV BamHI terminal fragment isolated from a gel, using standard procedures as described by T. Maniatis et al. "Molecular Cloning: A Laboratory Manual", New York, Cold Spring Harbor Laboratory (1982) and Holmes and Quigley, Analytical Biochemistry 114, 193-197. A recombinant plasmid pB3 was isolated.

### Cloning of NERs

The three genes under test for non-essentiality were retained together in their natural sequence in a shortened modification of plasmid pB3, see Figure 8. pB3 was shortened by removing the short unique sequence and the repeated sequences (see Figure 8) from the BamHI fragment. This was done by a digestion with SstI There is a SstI site in pBGS19 just before the BamHI site into which the FPV BamHI fragment was inserted. The SstI digestion was carried out at 37°C in a total of 20µl of the recommended buffer, using 10 units of SstI. There are many SstI sites in the repeated sequence, the last of which causes cutting at nucleotide 4005. Thus, after cutting with SstI, the plasmid pB3 (9.8kb) was re-ligated in a 100µl volume, to favour intramolecular ligation using the previously described ligation buffer, at 15°C overnight, to produce plasmid pB3ME (5.8kb) which lacks 4.0kb of the BamHI fragment DNA. To select the 5.8kb clones, rather than those which have lost shorter lengths of SstI- cut DNA, selected colonies were grown up and then subjected to gel electrophoresis. To check that all the repeats were removed, the plasmid was digested with BglII and the fragments analysed by gel electrophoresis.

### Preparation of a plasmid containing the VV 7.5K promoter and the lacZ gene

The VV 7.5K promoter is available in a plasmid pGS20 from Dr. Geoff Smith, Division of Virology, Dept, of Pathology, University of Cambridge, Addenbrooke's Site, Hills Road, Cambridge, England. Cambridge and is described in M. Mackett, G.L. Smith and B. Moss J. Virology 49, 857-864 (1984) and can be combined with E. coli. lacZ sequences as shown in the DNA sequence below. The resultant plasmid is designated pPG1. Other methods which could be used to combine the VV 7.5K promoter and lacZ sequence have been described in papers relating to VV.

<-----  p7.5 sequences from pGS20 ---------------------------
1 GGATCCCCAATTCCAGCTTGGCTGCAGGTCGACATATACTATATAGTAATACCAATACTC

------------------------------------------------------------
61 AAGACTACGAAACTGATACAATCTCTTATCATGTGGGTAATGTTCTCGATGTCGAATAGC

------------------------------------------------------------
121 CATATGCCGGTAGTTGCGATATACATAAACTGATCACTAATTCCAAACCCACCCGCTTTT

------------------------------------------------------------
181 TATAGTAAGTTTTTCACCCATAAATAATAAATACAATAATTAATTTCTCGTAAAAGTAGA

--------p7.5 sequences from pGS20 ------------------------>
241 AAATATATTCTAATTTATTGCACGGTAAGGAAGTAGAATCATAAAGAACGTGACGGATCG

<-SV40 sequences + linking DNA sequences ---->< -- lacZ sequences
301 AAGCTTTGCAAAGATGGATAAAGTTTTCCGGAATTCCAAGCTTGGCCTGGCCGTCGTTTT
MetAspLysValPheArgAsnSerLysLeuGlyLeuAlaValValLeu

------------------------------------------------------------
361 ACAACGTCGTGACTGGGAAAACCCTGGCGTTACCCAACTTAATCGCCTTGCAGCACATCC
GlnArgArgAspTrpGluAsnProGlyValThrGlnLeuAsnArgLeuAlaAlaHisPro

.................continuation of lacZ sequences ............

------------------------------------------------------------
3301 GGGGATTGGTGGCGACGACTCCTGGAGCCCGTCAGTATCGGCGGAATTCCAGCTGAGCGC
GlyIleGlyGlyAspAspSerTrpSerProSerValSerAlaGluPheGlnLeuSerAla

---- lacZ sequences ---------------------------------><--linking
3361 CGGTCGCTACCATTACCAGTTGGTCTGGTGTCAAAAATAATAATAACCGGGCAGGGGGGA
GlyArgTyrHisTyrGlnLeuValTrpCysGlnLys***

sequences--><---- SV40 sequences ------------------------------
3421 TCTGAACTTGTTTATTGCAGCTTATAATGGTTACAAATAAAGCAATAGCATCACAAATTT

------------------------------------------------------------
3481 CACAAATAAAGCATTTTTTTCACTGCATTCTAGTTGTGGTTTGTCCAAACTCATCAATGT

--SV40 sequences-->
3541 ATCTTATCATGTCTGGATCGGGGATCC

### Insertion of the VV 7.5K promoter/lacZ gene construct into FPV NERs to make recombination plasmids

The P7.5+lacZ fragment (about 3.6 kb) in 20µl total volume of buffer was cut out of the ampicillin - resistant plasmid pPG1 with 10 units of BamHI. The ends of the DNA were then repaired with the Klenow fragment of DNA polymerase to produce blunt-ended DNA molecules. Thus, the fragment was phenol-extracted with an equal volume of TE- saturated phenol and ethanol- precipitated. It was resuspended in 20µl of ligation buffer containing 5 units of Klenow fragment in 1 unit of T₄ polymerase, containing 0.025 mM of each of dATP, dCTP, dTTP and dGTP and stood for 1 hour at room temperature. Similarly, the pB3ME (SpeI, NcoI and BglII) were also digested with BamII and repaired to produce blunt-ended sites. The P7.5+lacZ fragment was then blunt-end ligated into pB3ME as follows. Thus, the plasmid and fragment (20 ng each) were ligated in a total volume of 10 µl above described ligation buffer containing 1 unit of T₄ DNA ligase and 1mM ATP and incubated at 4°C overnight and were transformed into E. coli. Blue colonies of X-gal Kanamycin plates were selected (pBGS 19 is a Kanamycin - resistant vector) and the DNA of several colonies was examined by the method of Holmes and Quigley, loc. cit. Each ligation produced two different results, i.e. the P7.5+lacZ could be ligated into pB3Me in either of two different orientations. The pairs of constructs of each site were called pEFL2/4, pEFL5/6 and pEFL7/8. In pEFL, 2, 6 and 8, the P7.5 + lacZ insert was in the same orientation as the gene into which it was inserted i.e. left to right in pEFL2 (BglII, 26.0) and pEFL8 (SpeI, 11.5) and right to left in pEFL6 (NcoI, 13.9).

### Recombination

The plasmids pEFL 2, 6 and 8 which contained the P7.5K promoter/lacZ construct in the orientations described were used in recombination experiments. The recombination has the following general stages:
1. Infect CEF cells with FPV, and add the recombination plasmid DNA in the form of a pre-prepared calcium phosphate precipitate. Allow to grow for several days. (Recombination takes place)
2. Harvest cells and freeze-thaw three times (Virus is released from cells).
3. Add this preparation to CEFs at various dilutions to produce plates with about 500-1000 plaques per 10cm dish (about 0.1% of these are recombinant plaques).
4. Add Xgal overlay and visualise blue plaques.
5. Pick isolated plaques. Freeze-thaw three times to release virus from cells of plaque.
6. Titrate again on CEFs and get more plaques.
7. Loop back to 4, repeating stages 4-6, until a pure plaque of the recombinant is obtained.

In more detail, chick embryo fibroblast cells, when about 80% confluent, were infected with about 3 plaque forming units (infectious virus particles) per cell of plaque-purified HP 440. fowlpoxvirus. The virus was contained in 1ml of commercially available serum free medium ("199"), and was left to adsorb to the cells for 2 hours at 37°C, after which the virus inoculum was removed and 10ml of fresh medium containing 5% newborn calf serum (CS) was added. 1.5 hours later a precipitate of the plasmid DNA construct to be recombined into the virus was prepared: to 1ml of Hepes-buffered saline (pH7.12) was added 10-20 µg of the pEFL 2, 6 or 8 plasmid DNA in solution in TE at about 1µg/µl. 50µl of 2.5M calcium chloride was added slowly, and a fine DNA/calcium phosphate precipitate was allowed to form for 30 minutes at room temperature (25°C). The medium from the virus-infected cells was then removed, the 1ml of precipitate was added to the cells and left for 30 minutes at room temperature. Then 10ml of fresh medium containing 5% CS was added to the cells, and they were incubated at 37°C for a further 4 hours. The condition of the cells was then examined and if the DNA calcium phosphate precipitate was considered too thick, the medium was replaced. Otherwise it was then replaced the next day. The cells were incubated at 37°C for a further 5 days and then the virus was harvested by scraping the cells into the medium. Intracellular virus was released by three cycles of freeze-thawing and the virus titrated by a conventional plaque titration.

The liquid medium was removed from the cells and an overlay of cell culture medium in 1% low gelling temperature agarose (LGT) containing 300µg/ml) of Xgal of (5-bromo-4-chloro-3-indolyl-beta D-galactoside) was added. After overnight incubation at 37°C, plaques expressing the lacZ gene, blue in colour, were observed.

Blue plaques were picked into 500 µl of 10mM Tris pH9, intracellular virus was released by three cycles of freeze-thawing and the virus titrated by a conventional plaque titration. The process of picking plaques and re-titrating was repeated until a plaque purified recombinant was obtained.

The plaque-purified recombinants were then passaged in tissue culture for the following numbers of passages after the first picking:

| | | |
|---|---|---|
| recombinants from plasmid pEFL | 8 | (SpeI insertion): stable for at least 3 passages |
| | 6 | (NcoI insertion): stable for only 2 passages |
| | 2 | (BglII insertion): stable for at least 6 passages. |

It is possible that the NcoI-based recombinants might be stable for a longer period under different conditions from those tried above.

### DNA analysis of FPV recombinants

When the DNA of the FPV parent is digested with BamHI a number of restriction fragments are generated, which can be separated on agarose gels. The terminal fragment is of size 6.3kb and is of double intensity compared with the other bands since there are two copies of it (one from each end of the genome). When the DNA of the recombinant made with pEFL2 was digested the pattern of bands was the same except that the double 6.3kb fragment had been replaced by a double 9.9kb fragment. This is consistent with the increase in size from 6.3kb to 9.9kb which would be produced if the 3.6kb P7.5+lacZ fragment had been inserted into the terminal fragment. Probing of the restriction digest after transfer to nitrocellulose showed that the 9.9kb band did in fact contain lacZ sequences.

### EXAMPLE 2

This Example relates to D8-D9 NERs.

In order to test whether D9, D10 and the fowlpox gene present in the D8 position were non-essential for growth further manipulations were carried out.
1. For D8 the plasmid pMB389 was grown in a dam⁻ E. coli host WK262 and reisolated as pMB434. The reason fro this is that in the usual strains of E. coli, the enzyme BclI to be used here cannot restrict because its control adenine base in its TGATCA recognition sequence is methylated. The dam⁻ strain resists methylation and in this host the enzyme BclI would be able to cleave its two recognition sites which occured in the D8 region. This plasmid was then cleaved with BclI and religated and a clone from which the 583 bp BclI fragment was missing was isolated by screening a number of colonies with small plasmid preps. Such a plasmid pMB441 containing a unique BclI site and having much of its D8 region removed was isolated. A BamHI fragment from pBPG1, containing the β-galactosidase gene, under the control of the vaccinia P7.5K promoter, was then inserted into this unique BclI site of pMB441 (BamHI and BclI generate compatible sticky ends such that no end-repairing was necessary). A plasmid clone pMB447 containing the correct structure was isolated and used in recombination assays (see later).
2. For D9 pMB389 was cleaved with AsuII (a unique site for which exists in the D9 gene) and end-repaired. pBPG1 was cleaved with BamHI and end-repaired and then ligated with AsuII cleaved pMB389 which had also been end-repaired. One colony selected as blue on Xgal Amp plates was found to contain the vaccinia P7.5K promoter and β-galactosidase gene inserted in the D9 gene.
3. For D10 an EcoRI fragment was subcloned from pMB389 (one EcoRI site lies within the D9 gene whilst the other is in the polylinker of the pUC13 vector of pMB389) into pUC13 cut with EcoRI. The resulting plasmid pMB439 contains a unique BglII site within the D10 gene. The BamHI fragment containing the vaccinia P7.5K promoter and the β-galactosidase gene were cloned into the BglII site of pMB439 to generate pMB443. (Again no end-repair was necessary as BamHI and BglII also have compatible sticky ends). Plasmid pMB443 was then tested in recombination assays.

The plasmid constructions described above are illustrated schematically in Fig. 10. The plasmid pBG1 is derived by cutting the BamHI fragment from pPG1, this fragment containing the P7.5-β-galactosidase gene construct, and inserting it in the plasmid pBGS19 (refer to Example 1).

### Recombination

Viruses from initial blue plaques from a recombination experiment were plaque-purified three times and appeared stable (i.e. no white plaques were observed on the third passage). Viral DNA was made from the plaque-purified viruses, and digested with EcoRI. DNA from the original (non-recombinant) ("wild type") FPV was also digested with EcoRI to serve as a control. The DNAs were run on duplicate agarose gels, Southern blotted, and probed with either the putatively recombinant FPV EcoRI fragment, within which the insertions should have taken place, or the pPG1 containing the β-galactosidase gene. Comparing the putatively recombinant FPV with the wild type when the EcoRI digests were run on gels, the normal EcoRI fragment present in wild-type DNA had increased in size in both the D8 and D9 putative recombinants. The new D8 fragment was smaller than the new D9 one, as expected, due to the removal of the small BclI fragment (377-960) in this case. When probed with β-galactosidase gene-containing pBG1 plasmid DNA, wild-type FPV DNA did not light up but the D8 and D9 putative recombinants both lit up in the same positions as when the probe from the putatively recombinant EcoRI fragment was used. This established that the structures of the D8 and D9 recombinants were as expected.

Similar experiments for the D10 gene failed to give the desired confirmations.

### EXAMPLE 3

### Virus strain.

This Example describes the construction of vectors containing Newcastle Disease Virus (NDV) F and HN genes, successful protection of birds using the F. gene. TIR NERs were used.

### A. Subcloning of NDV Fusion (F) and Haemagglutinin-Neuraminidase (HN) genes.

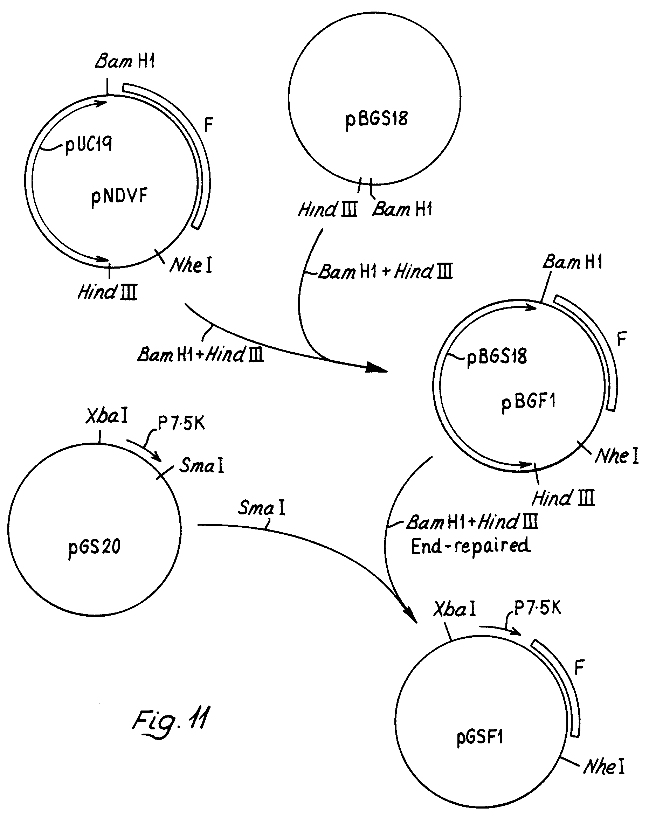

The F and HN genes of NDV were provided as a gift from Professor P.T. Emmerson of the University of Newcastle-upon-Tyne, each cloned into the plasmid pUC19, P. Chambers et al., Journal of General Virology, 67, 2685-2694 (1985); N.S. Millar et al., Journal of General Virology, 67, 1917-1927 (1986). See also European Patent Application 227414 (National Research Development Corporation) which refers to Budapest Treaty patent deposits of NDV cDNA in plasmids pUC18, and pUC19 at the National Collection of Industrial Bacteria, Aberdeen, Scotland on 1st July 1986 as NCIB 12277 (F gene) and 12278 (HN gene). Referring to the pUC19 clones actually used herein, the F gene clone also contained part of the HN gene downstream thereof. The F gene was subcloned by cutting with the restriction enzyme BamHI (which conveniently cuts 29 bases upstream of the initiating ATG codon at the 5′ end of the F gene) and HindIII (which cuts approximately 820 bases downstream from the 3′ end of the F gene, this extra sequence including some of the HN gene) into the kanamycin- resistant plasmid pBGS18 cut with BamHI and HindIII. The resulting plasmid was called pBGF1 (see Figure 11).

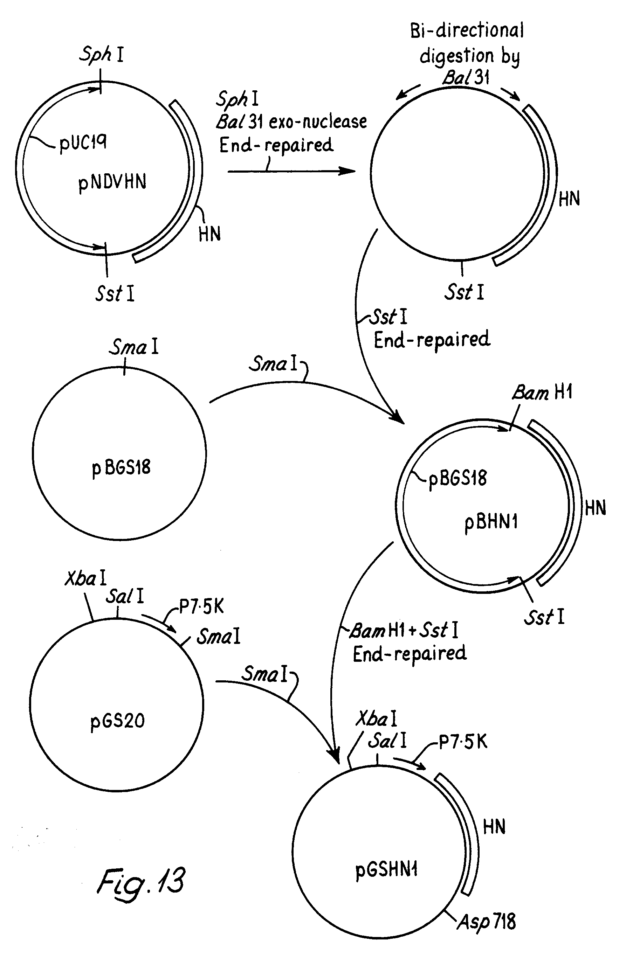

The pUC19 plasmid containing the HN gene also has F gene sequence upstream of the HN gene. In order to facilitate positioning of a poxvirus promoter adjacent to the HN gene, the F gene sequences were removed by the use of the exonuclease Bal31. The plasmid was cleaved with the restriction enzyme SphI (which cuts in the mutliple cloning site of pUC19 on the 5′ side of the HN gene) and digested for a range of appropriate times as follows. The method was as described in "Molecular Cloning (A Laboratory Manual)" by T. Maniatis et al., Cold Spring Harbor USA (1982). 20µl of DNA (at approximately 500µg/ml) were mixed with 20µl BSA (1mg/ml) and 40µl of 2 x "Bal31 buffer" (24mM CaCl₂, 24mM MgCl₂, 0.4M NaCl, 40mM Tris/HCl pH 8.0, 2mM EDTA) and warmed to 30°C. 0.5 unit of Bal31 was added and samples were incubated for 0, 1, 2, 3, 4 and 5 minutes. Then 20 µl of water and 3µl of 0.5M Tris/HCl pH 7.5, 100mM MgCl₂ were added, and the samples were digested with 10 units of SstI (which cuts downstream of the HN gene) for 60 minutes. Samples were run on an agarose gel to estimate the amount of DNA digested, and the 0, 1 and 2 minute samples were chosen for further use. The molecules were then repaired (as described above) with T4 DNA polymerase and Klenow fragment and cloned into SmaI-cut pBGS18. Several different isolates were sequenced at the 5′ end of the HN gene and one was found in which only 49 of the bases upstream of the gene remained (starting at the sequence GGCTTCACAA...). This plasmid was called pBHN1 (see Figure 13).

### B. Cloning of the F gene into a recombination vector.

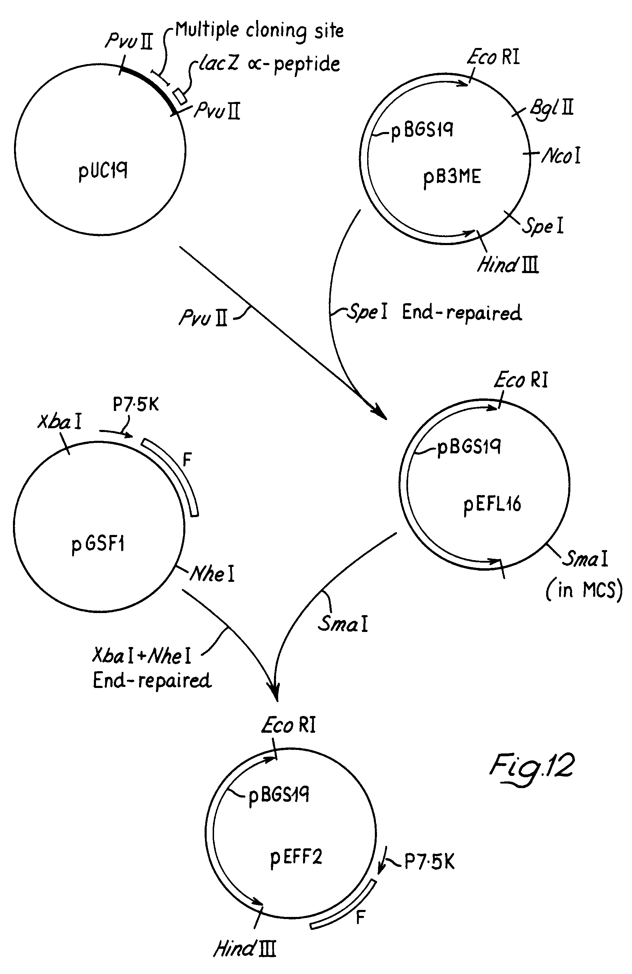

In order to place the F gene under the control of the P7.5K VV promoter, the F gene was excised from pBGF1 with BamHI and HindIII, end-repaired as described, and cloned into the SmaI site of pGS20 (Mackett et al., Journal of Virology 49, 857-864). This plasmid was called pGSF1 (see Figure 11). A plasmid pEFL16 (Figure 12) was constructed by inserting the PvuII fragment from pUC19 into the SpeI site of pB3Me (described above). This fragment contains the coding sequences of the alpha peptide of beta-galactosidase with a multiple cloning site arranged such that when fragments are cloned into this site the translation of the beta-galactosidase is disrupted and thus recombinants can be detected because they are white colonies on plates containing Xgal. (This construct pEFL16 is now equivalent to the recombination plasmid' described in Example 1 and shown in Figure 7). The F gene plus P7.5 promoter was excised from pGSF1 by using XbaI and NheI, end-repaired as described, and cloned into the unique SmaI site of pEF16. The resulting plasmid (which now has the F gene, with the P7.5 promoter upstream, placed within the SpeI non-essential region of the terminal fragment of fowlpox) is called pEFF2 (Figure 12).

### C. Cloning of the HN gene into a recombination vector.

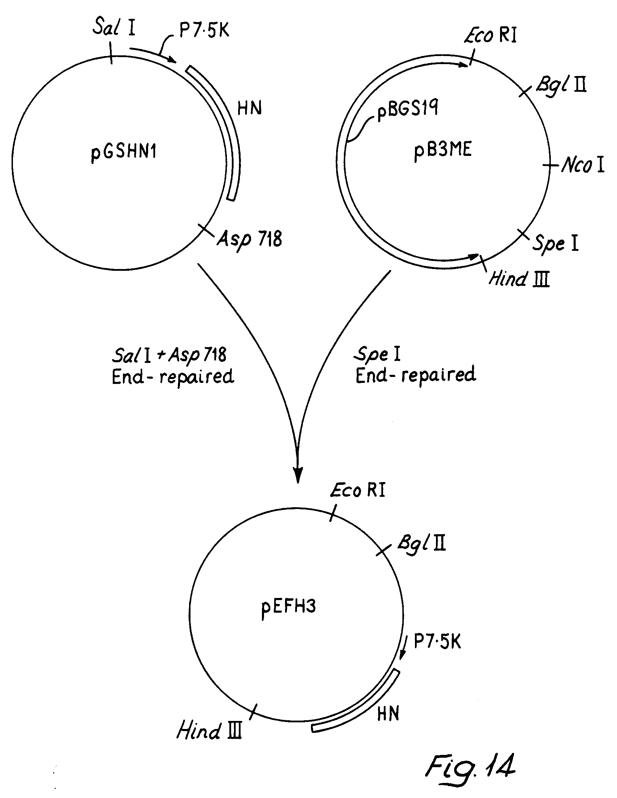

In order to place the HN gene under the control of the P7.5 promoter it was excised from pBHN1 with SstI and BamHI, repaired as described, and cloned into the SmaI site of pGS20. This plasmid was called pGSHN1 (Figure 13). The HN gene plus P7.5 promoter was excised from pGSHN1 by using SalI and Asp718, repaired as described, and cloned into pB3ME which had been cut with SpeI and repaired. This plasmid was called pEFH3 (see Figure 14).

### D. Production of FPV recombinant containing the F gene.

The Poxine strain of fowlpox virus used was kindly donated by Duphar. It was passaged three times in chicken embryo fibroblast cells CEFs in tissue culture. A single plaque was picked at this stage and was plaque-purified three times. The virus thus obtained was called Px4.1. and was grown in tissue culture as described for the HP444 strain in Example 1.

The plasmid pEFF2 was allowed to recombine into the Px4.1 strain of FPV, as described in Example 1. The virus from the recombinations was titrated on CEFs and then plaqued out on 10cm petri dishes at a concentration of virus producing about 1000 plaques per dish. The virus was overlaid with cell culture medium in 1% low gelling temperature agarose. After 4-5 days the agarose overlay was removed and stored at 4°C in a sterile petri dish. A dry nitrocellulose filter was laid onto the cell sheet and pressed down with a circle of Whatman 3MM chromotography paper soaked in 20 X SSC. The filter was then lifted off the dish, with the cell sheet and plaques adhered to it, the 3MMs paper removed, and the filter was baked at 80°C in a vacuum for 2 hours. To identify recombinant viruses, the filters were then probed with radiolabelled probes specific for either the F gene or the HN gene. The filters were exposed to X-ray film and the position of recombinant plaques identified by aligning the autoradiograms with the stored agarose overlays. Recombinant virus was then isolated from the agarose overlay by removing a plug of agarose using a pasteur pipette. This plug was resuspended in 10mM Tris pH 9.0, freeze-thawed three times and replaqued. Recombinant virus was identified again by probing, and this process was repeated three times in all until a plaque-purified virus was achieved.

### E. Production of FPV recombinant containing the HN gene.

The plasmid pEFH3 was allowed to recombine, as described above, into the plaque-purified HP440 virus (HP 438 as described in Example 1, plus two further passages). This virus is called FP9. Recombinant virus containing the HN gene was isolated as described for the F gene.

### F. Expression of the F gene in tissue culture.

The recombinant viruses, and the equivalent parent strain as controls, were used to infect 5cm petri dishes of CEFs at approximately 5 pfu/cell. The virus was allowed to grow for various times and then the cells lysed by addition of 500µl of boiling sample buffer (containing 24mM Tris/HCl pH 6.8 1% SDS, 20% glycerol and 0.02% bromophenol blue). For the HN recombinant this buffer also included 0.1M dithiothreitol. The infected cells were scraped into the sample buffer, and the sample boiled for 2 minutes, cooled on ice, and frozen at -20°C. Samples were then loaded onto standard polyacrylamide protein gels, electrophoresed to separate the proteins, and transferred to nitrocellulose filters by Western blotting. The Western blots were probed (as described in A.C.R. Samson et al., Journal of General Virology 67, 1199-1203) with a monoclonal antibody specific to the F protein or the HN protein did not reveal any F protein produced by the recombinant. The F protein produced by infection of CEFs with NDV was detected by the monoclonal antibody, but at a lower level than the HN, and so if the same ratio of native to recombinant protein held for the F as for the HN, the recombinant F might indeed not be seen.

### G. Protection of birds against NDV challenge

14 days old Rhode Island Red chickens were inoculated intravenously with 10⁶ pfu/bird of the F gene recombinant (4 birds) or of a fowlpox strain FP9 having no inserted genes (6 birds). 6 birds were left uninoculated. The 10 birds not inoculated with the recombinant are hereinafter referred to as 'the control birds'. At 28 days the inoculation was repeated. At 64 days the birds were challenged intramuscularly with 10⁵ x ELD₅₀ (ELD = embryo lethal dose) of the virulent Herts 33 strain of NDV. At 3 days after challenge, 4 of the control birds were dead and 8 were very sick. At 4 days after challenge, 10 out of 12 control birds were dead, and by 5 days after challenge all the control birds were dead. All 4 birds inoculated with the F recombinant were normal.

Serum was taken from birds inoculated with the F recombinant and control birds before challenge with NDV. Examination of this serum by probing Western blots of purified NDV virions, showed that only the serum from the birds inoculated with the F recombinant had antibodies to the F protein. Thus although it was not possible to detect F protein produced by the recombinant in vitro, the in vivo experiment showed very convincingly that protection could be achieved, and that F protein in some form has been produced by the FPV recombinant containing the NDV F gene.

Example 6 describes a protection experiment using the NDV HN gene constructs of this Example.

### EXAMPLE 4

This Example demonstrates vaccination of birds at one day of age with an NDV F gene/ FPV recombinant.

Groups of six or seven Rhode Island Red chicks were inoculated, at one day of age, by the wing-web method with either the NDV F gene/poxine FPV recombination described in Example 3 8907374 or the Px4.1 parent poxine FPV strain. Each virus strain was inoculated into four groups of birds, at doses of 5 x 10⁴, 5 x 10³, 5 x 10², and 5 x 10¹ pfu/bird. Thus there were eight groups in all, with an additional group of uninoculated birds. Birds were inoculated at one day of age and challenged intramuscularly 26 days later, as in Application 8907374. Table 1 shows the results of this challenge. Uninoculated and control birds were not protected against challenge, all 38 birds being dead by 4 days. The birds inoculated with the fowlpox/NDV F recombinant were protected to a significant degree, 22 birds out of 28 surviving the challenge (79%). The dose of vaccine used appeared to have little effect on the levels of protection, although the lowest dose gave the best protection.

**TABLE 1**

| Inoculum | Viral dose | Total number of birds dead at days 3-6 after challenge with NDV Herts 33 strain | | | |
|---|---|---|---|---|---|
| | | days 3 | day 4 | day 5 | day 6 |
| NDV F gene/Px4.1 FPV recombinant | 5 x 10⁴ | 0/7 | 0/7 | 2/7 | 2/7 |
| | 5 x 10³ | 0/7 | 0/7 | 2/7 | 2/7 |
| | 5 x 10² | 1/7 | 1/7 | 2/7 | 2/7 |
| | 5 x 10 | 0/7 | 0/7 | 0/7 | 0/7 |
| Px4.1 FPV alone | 5 x 10⁴ | 6/7 | 7/7 | | |
| | 5 x 10³ | 3/6 | 6/6 | | |
| | 5 x 10² | 5/6 | 6/6 | | |
| | 5 x 10 | 1/5 | 5/5 | | |
| Uninoculated | - | 4/7 | 7/7 | | |

### EXAMPLE 5

This Example confirms the results given in Example 3 (Section G) for vaccination of birds with an NDV F gene recombinant and shows that it can be done by using the wing web as well as the intravenous route.

The procedure of Example 3 was followed, except that the booster inoculation was at 27 days from birth (rather than 28) and the challenge at 42 days from birth (rather than 64), the parent poxine strain was used as control, and there were five groups of birds (two inoculated iv, two wing web and one uninoculated).

The results in Table 2 show that all the birds inoculated with the NDV F gene/poxine FPV recombinant survived, whereas all the others died.

**TABLE 2**

| Inoculum | Method | Total number of birds dead at days 3, 4 and 14 after challenge with NDV Herts 33 strain | | |
|---|---|---|---|---|
| | | day 3 | day 4 | day 14 |
| NDV F gene/Px4.1 FPV recombinant | iv | 0/6 | 0/6 | 0/6 |
| Px4.1 FPV alone | iv | 6/6 | | |
| NDV F gene/Px4.1 FPV recombinant | wing-web | 0/6 | 0/6 | 0/6 |
| Px4.1 FPV alone | wing-web | 4/6 | 6/6 | |
| Uninoculated | - | 9/13 | 13/13 | |

### EXAMPLE 6

This Example shows that a NDV HN gene/FPV recombinant also gives protection. Thus, protection can be obtained from either the F or the HN gene alone.

Example 5 was repeated except that the NDV HN/HP 440 FPV recombinant as described in Example 3 (Section E) was used with the HP 440 strain as control.

Table 3 shows the results. Again, the NDV HN gene-vaccinated birds were protected while the others all died.

**TABLE 3**

| Inoculum | Method | Total number of birds dead at days 3, 4 and 14 after challenge with NDV Herts 33 strain | | |
|---|---|---|---|---|
| | | day 3 | day 4 | day 14 |
| NDV HN gene/HP 440 FPV recombinant | iv | 1/6 | 1/6 | 1/6 |
| HP 440 alone | iv | 6/6 | | |
| Uninoculated | - | 9/13 | 13/13 | |

### EXAMPLE 7

In this Example recombination vectors were made using the Ecogpt gene, the constructs being of a generally similar kind to those described by CSIRO in the already cited PCT Application WO 88/02022, but flanked by non-essential region sequences of the present invention rather than TK gene sequences. The recombination vectors comprise in order:
(1) Non-essential region (around the BglII site)
(2) Ecogpt gene
(3) VV P.5 promoter
(4) VV P11 promoter
(5) IBV spike protein gene
(6) Non-essential region.

Note that the two promoters are "back-to-back", as shown in WO 88/02022, transcribing in opposite direction.

The clone pEFL2 (containing the vaccinia P7.5 promoter and beta-galactosidase gene in the FPV terminal fragment, see above) was cut with the restriction enzymes SphI and Nco1, repaired as described and religated. The resulting construct, designated pEFL18, had lost approximately 850 base pairs of FPV sequence, as expected, but had also lost a HindIII site immediately adjacent to the SphI site. Thus, this plasmid contains only two HindIII sites, very close together, between the P7.5 promoter and the beta-galactosidase gene. (Digestion with HindIII thus only removes a very small fragment and can be used for the insertion of gene fragments containing their own ATG codon to make beta-galactosidase fusion constructs). This plasmid was cut with HindIII and Esp1 (Esp1 cuts near to the 3′ end of the beta-galactosidase gene) and repaired. Into this repaired site was inserted a BglII/ApaI fragment (repaired) from the plasmid pSV2gpt (Mulligan & Berg, Science 209, 422-1427, 1980) which contained the E. coli ecogpt gene. Thus, the beta-galactosidase gene was removed and the Ecogpt gene was placed under the control of the P7.5 promoter. A unique SalI site upstream of the promoter allows insertion of other promoters or promoter/gene constructs. This plasmid was called pEFGP13.

The P11 late vaccinia promoter was excised from pMM6 (a gift from Michael Mackett) provided with multiple cloning sites (BamHI, XhoI, XbaI and SalI) by transfer to pUC19 and the resultant P11-cloning site construct inserted into the SalI site of pEFGPT3. The resultant plasmid was designated pEFGPT6 and allows insertion of genes at various sites under the control of the P11 promoter. An infectious Bronchitis Virus spike protein gene (see NRDC's PCT Application Publication No. WO86/05806) was inserted downstream of the promoter.

The plasmid pEFGPT3 was allowed to recombine into FPV strain FP9, as described above. Recombinant viruses were selected by the use of MXHAT medium containing mycophenolic acid (Boyle & Coupar, Gene 65, 123-129, 1988). After the initial recombination in the presence of MXHAT medium, the virus was released by freeze-thawing and passaged twice under selective conditions. A cytopathic effect was seen, indicating that virus was growing under the selective conditions. Virus was then plaqued out and probed with an Ecogpt-specific probe. A high proportion of plaques (approximately 75%) were positive, indicating that the selection had been successful (normally only 0.1% of the virus would be recombinants) and hence that recombinants expressing the Ecogpt gene had been isolated.

Similar recombination experiments were carried out to incorporate the Ecogpt gene into the Poxine strain of FPV. After the recombination and upon passage in selective conditions, a cytopathic effect of the virus could be seen, indicating that recombinant virus expressing the Ecogpt gene had been produced. This confirms that the BglII non-essential region (as well as the SpaI region) is present in Poxine.

## Claims (Claims for the following Contracting State(s): BE, CH, LI, DE, FR, IT, NL, SE)

1. A recombination vector for use in producing a recombinant fowl pox virus (FPV) by homologous DNA recombination, said recombination vector comprising a cloning vector containing a non-essential region (NER) DNA sequence, said NER being interrupted by at least one foreign gene and by at least one sequence for regulating the expression of the or each foreign gene, characterised in that the NER sequence is (a) within the long unique sequence (LUS) of the terminal inverted repeat (TIR) of FPV or (b) within the region of the FPV genome lying between the carboxy-end of the D7 gene and the carboxy-end of the D9 gene (but excepting the D10 gene and D10 gene promoter region).

2. A recombination vector according to Claim 1, characterised in that the NER lies wholly within 2.2 kilobase pairs from the external end of the LUS.

3. A recombination vector according to Claim 1 or 2, characterised in that the NER lies within an open-reading frame (ORF) which is substantially one of those shown at 4125-4790, 5261-5629 or 5883-6185 in Sequence No. 1 and having the nucleic acid sequence therein identified or a variant thereof occurring in another strain of FPV.

4. A recombination vector according to Claim 1, characterised in that the NER lies within the D8 or D9 gene.

5. A recombination vector according to Claim 1, characterised in that it comprises in order :
(1) a first homologously recombinable flanking sequence,
(2) a sequence within a first portion of the non-essential region,
(3) promoter DNA,
(4) a foreign gene transcribably downstream of the promoter (whereby when the fowl pox virus RNA polymerase binds to the promoter it will transcribe the foreign gene into mRNA),
(5) a sequence within a second portion of the same non-essential region, the first and second sequences being in the same relative orientation as are the first and second portions of the non-essential region within the viral genome, and
(6) a second homologously recombinable flanking sequence,

6. A recombinant vector useful in preparing a recombination vector according to Claim 1, this recombinant vector being characterised by containing a NER sequence as defined in Claim 1,2,3, or 4.

7. A DNA molecule which consists essentially of a NER sequence as defined in Claim 1,2,3,4 or 5.

8. A recombinant fowlpox virus (FPV) which is the product of homologous recombination of a parent FPV with the insert DNA of a recombination vector claimed in any one of Claims 1-5.

9. An in vitro culture of animal cells infected with a recombinant FPV according to Claim 8.

10. A recombinant FPV according to Claim 8 or culture according to Claim 9, for use in vaccinating an animal.

11. A recombinant FPV or culture according to Claim 10 wherein the animal is a chicken.

## Claims (Claims for the following Contracting State(s): ES)

1. A process of preparing a recombination vector for use in producing a recombinant fowl pox virus (FPV) by homologous DNA recombination, in which (1) one introduces a non-essential region (NER) DNA sequence into a cloning vector and one interrupts the NER by introducing thereinto at least (2) one foreign gene and at least one sequence for regulating the expression of the or each foreign gene, characterised in that as the NER sequence one uses DNA (a) within the long unique sequence (LUS) of the terminal inverted repeat (TIR) of FPV or (b) within the region of the FPV genome lying between the carboxy-end of the D7 gene and the carboxy-end of the D9 gene (but excepting the D10 gene and D10 gene promoter region).

2. A process according to Claim 1, characterised in that one introduces the foreign gene into the multiple cloning site of a cloning vector containing in order:
(1) a first homologously recombinable flanking sequence,
(2) a sequence within a first portion of the non-essential region,
(3) promoter DNA,
(4) a multiple cloning site downstream of the promoter,
(5) a sequence within a second portion of the same non-essential region, the first and second sequences being in the same relative orientation as are the first and second portions of the non-essential region within the viral genome, and
(6) a second homologously recombinable flanking sequence.

3. A process according to Claim 1 or 2, characterised in that the NER lies wholly within 2.2 kilobase pairs from the external end of the LUS.

4. A process according to Claim 3 characterised in that the NER lies within an open-reading frame (ORF) which is substantially one of those at 4125-4790, 5261-5629 or 5883-6185 in Sequence No. 1 and having the nucleic acid sequence therein identified or a variant thereof occurring in another strain of FPV.

5. A process according to Claim 1 or 2, characterised in that the NER lies within the D8 or D9 gene.

6. A process of preparing a recombinant vector useful in preparing a recombination vector, characterised in that one introduces a NER sequence consisting essentially of one as defined in Claim 1,3,4 or 5, into a cloning vector.

7. A process of preparing a recombinant fowl pox virus by infecting animal cells in vitro with a parent, infecting strain of fowlpox virus, introducing DNA of the recombination vector into said cells and allowing homologous recombination to take place, characterised in that a recombination vector prepared by a process according to any one of Claims 1-5 is used.

## Patentansprüche (Patentansprüche für folgende(n) Vertragsstaat(en): BE, CH, DE, FR, IT, LI, NL, SE)

1. Rekombinanter Vektor zur Verwendung bei der Herstellung eines rekombinanten Fowlpoxvirus (FPV) durch homologe DNA-Rekombination, wobei der Rekombinationsvektor einen Klonierungsvektor umfaßt, der eine DNA-Sequenz aus einer nichtessentiellen Region (NER) enthält, wobei die NER durch mindestens ein Fremdgen und mindestens eine Sequenz zur Expressionsregulation des Fremdgens oder aller Fremdgene unterbrochen ist, dadurch gekennzeichnet, daß die NER-Sequenz (a) innerhalb der long-unique Sequenz (LUS) der terminal-inverted-repeat Einheit (TIR) des FPV oder (b) innerhalb der Region zwischen dem Carboxyende des D7-Gens und dem Carboxyende des D9-Gens (jedoch unter Ausnahme des D10-Gens und der D10-Genpromotorregion) liegt.

2. Rekombinationsvektor nach Anspruch 1, dadurch gekennzeichnet, daß die NER vollständig innerhalb von 2,2 Kilobasenpaaren vom äußeren Ende des LUS liegt.

3. Rekombinationsvektor nach Anspruch 1 oder 2, dadurch gekennzeichnet, daß die NER innerhalb eines offenen Leserasters (ORF), welcher im wesentlichen einer derjenigen wie in der Sequenz Nr. 1 bei 4125-4790, 5261-5629 oder 5883-6185 gezeigt, ist, und die darin identifizierte Nukleinsäuresequenz hat, oder eine Variante davon, die in einem anderen FPV-Stamm vorkommt.

4. Rekombinationsvektor nach Anspruch 1, dadurch gekennzeichnet, daß die NER innerhalb des D8- oder D9-Gens liegt.

5. Rekombinationsvektor nach Anspruch 1, dadurch gekennzeichnet, daß er in der folgenden Reihenfolge enthält:
(1) eine erste homolog rekombinierbare flankierende Sequenz,
(2) eine Sequenz innerhalb eines ersten Teils der nichtessentiellen Region,
(3) Promotor-DNA,
(4) ein Fremdgen, das stromabwärts des Promotors transkribierbar ist (wodurch bei Bindung der Fowlpoxvirus-RNA-Polymerase am Promotor das Fremdgen in mRNA transkribierbar wird),
(5) eine Sequenz innerhalb eines zweiten Teils derselben nichtessentiellen Region, wobei die erste und zweite Sequenz dieselbe relative Orientierung wie der erste und zweite Teil der nichtessentiellen Region innerhalb des viralen Genoms aufweisen, und
(6) eine zweite homolog rekombinierbare flankierende Sequenz.

6. Rekombinanter Vektor, der zur Herstellung eines Rekombinationsvektors nach Anspruch 1 geeignet ist, wobei dieser rekombinante Vektor dadurch gekennzeichnet ist, daß er eine NER-Sequenz wie in Anspruch 1, 2, 3 oder 4 definiert, umfaßt.

7. DNA-Molekül, welches im wesentlichen aus einer NER-Sequenz wie in Anspruch 1, 2, 3, 4 oder 5 definiert, besteht.

8. Rekombinanter Fowlpoxvirus (FPV), welcher das Produkt einer homologen Rekombination eines Ausgangs-FPV's mit einer eingefügten DNA aus einem Rekombinationsvektor, wie in einem der Ansprüche 1 bis 5 beansprucht, ist.

9. In vitro-Kultur von mit einem rekombinanten FPV nach Anspruch 8 infizierten tierischen Zellen.

10. Rekombinantes FPV nach Anspruch 6 oder Kultur nach Anspruch 9, zur Verwendung bei der Impfung eines Tieres.

11. Rekombinanter FPV oder Kultur nach Anspruch 10, worin das Tier ein Huhn ist.

## Patentansprüche (Patentansprüche für folgende(n) Vertragsstaat(en): ES)

1. Verfahren zur Herstellung eines Rekombinationsvektors zur Verwendung bei der Herstellung eines rekombinanten Fowlpoxvirus (FPV) durch homologe DNA-Rekombination, bei dem man (1) eine DNA-Sequenz aus einer nichtessentiellen Region (NER) in einen Klonierungsvektor einführt und man die NER durch Einführung hierin von mindestens (2) einem Fremdgen und mindestens einer Sequenz zur Regulation der Expression des Fremdgens oder aller Fremdgene unterbricht, dadurch gekennzeichnet, daß man als NER-Sequenz eine DNA (a) innerhalb der long-unique Sequenz (LUS) der terminal-inverted-repeat Einheit (TIR) des FPV oder (b) innerhalb der Region des FPV-Genoms, die zwischen dem Carboxyende des D7-Gens und dem Carboxyende des D9-Gens liegt (jedoch unter Ausnahme des D10-Gens und der D10-Genpromotorregion), verwendet.

2. Verfahren nach Anspruch 1, dadurch gekennzeichnet, daß man das Fremdgen in die multiple Klonierungsstelle eines Klonierungsvektors einführt, der in der folgenden Reihenfolge enthält:
(1) eine erste homolog rekombinierbare flankierende Sequenz,
(2) eine Sequenz innerhalb eines ersten Teils der nichtessentiellen Region,
(3) Promotor-DNA,
(4) eine multiple Klonierungsstelle stromabwärts des Promotors,
(5) eine Sequenz innerhalb eines zweiten Teils derselben nichtessentiellen Region, wobei die erste und zweite Sequenz dieselbe relative Orientierung wie der erste und zweite Teil der nichtessentiellen Region innerhalb des viralen Genoms aufweist, und
(6) eine zweite homolog rekombinierbare flankierende Sequenz.

3. Verfahren nach Anspruch 1 oder 2, dadurch gekennzeichnet, daß die NER vollständig innerhalb von 2,2 Kilobasenpaaren vom äußeren Ende des LUS liegt.

4. Verfahren nach Anspruch 3, dadurch gekennzeichnet, daß die NER innerhalb eines offenen Leserasters (ORF), welcher im wesentlichen einer derjenigen bei 4125-4790, 5216-5629 oder 5883-6185 in Sequenz Nr. 1 ist, liegt, und die darin identifizierte Nukleinsäuresequenz oder eine Variante davon hat, die in einem anderen Stamm von FPV vorkommt.

5. Verfahren nach Anspruch 1 oder 2, dadurch gekennzeichnet, daß die NER innerhalb des D8- oder D9-Gens liegt.

6. Verfahren zur Herstellung eines rekombinanten Vektors, der zur Herstellung eines Rekombinationsvektors geeignet ist, dadurch gekennzeichnet, daß man eine NER-Sequenz, die im wesentlichen aus der, wie in Anspruch 1, 3, 4 oder 5 definiert, besteht, in einen Klonierungsvektor einführt.

7. Verfahren zur Herstellung eines rekombinanten Fowlpoxvirus durch Infektion von Tierzellen in vitro mit einem infektiösen Ausgangsstamm eines Fowlpoxvirus, Einführen der DNA des rekombinanten Vektors in die Zellen und Durchführung einer homologen Rekombination, dadurch gekennzeichnet, daß ein Rekombinationsvektor verwendet wird, hergestellt durch ein Verfahren nach einem der Ansprüche 1 bis 5.

## Revendications (Revendications pour l'(les) Etat(s) contractant(s) suivant(s): BE, CH, DE, FR, IT, LI, NL, SE)

1. Un vecteur de recombinaison à utiliser dans la production d'un virus de variole aviaire (V A) recombinant par recombinaison homologue d'ADN, ce, vecteur de recombinaison comprenant un vecteur de clonage contenant une séquence d'ADN de région non essentielle (RNE), ladite RNE étant interrompue par au moins un gène étranger et par au moins une séquence destinée à réguler l'expression du ou de chaque gène étranger, caractérisé en ce que la séquence RNE est (a) incluse dans la longue séquence unique (LSU) de la répétition inversée terminale (RIT) du VVA ou (b) incluse dans la région du génome de VVA qui se situe entre la terminaison carboxyle du gène D7 et la terminaison carboxyle du gène D9 (mais à l'exception du gène D10 et de la région du promoteur du gène D10).

2. Un vecteur de recombinaison selon la Revendication 1, caractérisé en ce que la RNE est entièrement incluse dans des paires de 2,2 kilobases à partir de la terminaison extérieure de la LSU.

3. Un vecteur de recombinaison selon la Revendication 1 ou 2, caractérisé en ce que la RNE est incluse dans un cadre de lecture ouverte (CLO) qui est pratiquement l'un de ceux représentés à 4125-4790, 5261-5629 ou 5883-6185 dans la Séquence n^{o} 1 et dont la séquence acide nucléique qui y est identifiée, ou une variante de celle-ci, se retrouve dans une autre souche de VVA

4. Un vecteur de recombinaison selon la Revendication 1, caractérisé en ce que la RNE est incluse dans le gène D8 ou D9.

5. Un vecteur de recombinaison selon la Revendication 1, caractérisé en ce qu'il comprend dans l'ordre :
(1) une première séquence voisine recombinable de façon homologue,
(2) une séquence incluse dans une première portion de la région non essentielle.
(3) un ADN activateur,
(4) un gène étranger situé de façon transcriptible en aval du promoteur (grâce auquel, lorsque le polymérase de l'ADN du virus de la variole aviaire est lié au promoteur , il transcrit le gène étranger en mRNA),
(5) une séquence incluse dans une seconde portion de la même région non essentielle, les premières et secondes séquences ayant la même orientation relative que les premières et secondes portions de la région non essentielle dans le génome viral, et
(6) une seconde séquence voisine recombinable de façon homologue.

6. Un vecteur recombinant utile dans la préparation d'un vecteur de recombinaison selon la Revendication 1, ce vecteur recombinant étant caractérisé en ce qu'il contient une séquence de RNE selon la définition donnée dans la Revendication 1, 2, 3 ou 4.

7. Une molécule d'ADN qui est essentiellement constituée d'une séquence de RNE selon la définition donnée dans la Revendication 1, 2, 3, 4 ou 5.

8. Un virus de variole aviaire (VVA) recombinant qui est le produit de la recombinaison homologue d'un VVA père avec l'ADN introduit d'un vecteur de recombinaison selon l'une des Revendications 1 à 5.

9. Une culture in vitro de cellules animales infectées par un VVA recombinant selon la Revendication 8.

10. Un VVA recombinant selon la Revendication 8 ou une culture selon la Revendication 9, à utiliser pour la vaccination d'un animal.

11. Un VVA recombinant ou une culture selon la Revendication 10 où l'animal est un poulet.

## Revendications (Revendications pour l'(les) Etat(s) contractant(s) suivant(s): ES)

1. Un procédé de préparation d'un vecteur de recombinaison à utiliser dans la production d'un virus de variole aviaire (VVA) recombinant par recombinaison homologue d'ADN, dans lequel (1) on introduit une séquence de région non essentielle (RNE)^{V} dans un vecteur de clonage et on interrompt la RNE en y introduisant au moins (2) un gène étranger et au moins une séquence destinée à réguler l'expression du ou de chaque gène étranger, caractérise en ce que la première séquence RNE utilise de l'ADN (a) dans la longue séquence unique (LSU) de la répétition inversée terminale (RIT) du VVA ou (b) dans la région du génome de VVA qui se situe entre la terminaison carboxyle du gène D7 et la terminaison carboxyle du gène D9 (mais à l'exception du gène D10 et de la région du promoteur du gène D10).

2. Un procédé selon la Revendication 1, caractérisé en ce que l'on introduit le gène étranger dans le site de clonage multiple d'un vecteur de clonage contenant dans l'ordre :
(1) une première séquence voisine recombinable de façon homologue,
(2) une séquence incluse dans une première portion de la région non essentielle,
(3) un ADN de promoteur,
(4) un site de clonage multiple en aval du promoteur,
(5) une séquence incluse dans une seconde portion de la même région non essentielle, les premières et secondes séquences ayant la même orientation relative que les premières et secondes portions de la région non essentielle dans le génome viral, et
(6) une seconde séquence voisine recombinable de façon homologue.

3. Un procédé selon la Revendication 1 ou 2, caractérisé en ce que la RNE est entièrement incluse dans des paires de 2,2 kilobases à partir de la terminaison extérieure de la LSU.

4. Un procédé selon la Revendication 3, caractérisé en en ce que le RNE est incluse dans un cadre de lecture ouverte (CLO) qui est pratiquement l'un de ceux situés à 4125-4790, 5261-5629 ou 5883-6185 dans la Séquence n^{o} 1, et dont la séquence acide nucléique est identifiée ou dont une variante se retrouve dans une autre souche de VVA.

5. Un procédé selon la Revendication 1 ou 2, caractérisé en ce que la RNE est incluse dans le gène D8 ou D9.

6. Un procédé de préparation d'un vecteur recombinant utile dans la préparation d'un vecteur de recombinaison, caractérisé en ce que l'on introduit dans un vecteur de clonage une séquence de RNE essentiellement constituée selon la Revendication 1, 3, 4 ou 5.

7. Un procédé de préparation d'un virus de variole aviaire recombinant en infectant des cellules animales in vitro avec une souche-mère de virus de diphtérie aviaire, en introduisant l'ADN du vecteur de recombinaison dans lesdites cellules et en permettant la réalisation d'une recombinaison homologue, caractérisé en ce qu'un vecteur de recombinaison préparé par un procédé selon l'une des Revendications 1 à 5 est utilisé.
